# EUROPEAN PATENT SPECIFICATION

(11) **EP 4 185 693 B1**
(45) Date of publication and mention of the grant of the patent: **11.02.2026**
(21) Application number: 21749577.9
(22) Date of filing: 23.07.2021
(51) Int. Cl.: C12N 15/113, A61K 31/712, A61P 9/00

(54) **COMBINATORIAL INHIBITION OF TRANSCRIPTION FACTORS FOR TREATMENT OF HEART FAILURE**
KOMBINATORISCHE HEMMUNG VON TRANSKRIPTIONSFAKTOREN ZUR BEHANDLUNG VON HERZVERSAGEN
INHIBITION COMBINATOIRE DE FACTEURS DE TRANSCRIPTION POUR LE TRAITEMENT DE L'INSUFFISANCE CARDIAQUE

(30) Priority: 23.07.2020 EP 20187306; 21.01.2021 EP 21152752
(43) Date of publication of application: 31.05.2023
(73) Proprietor: Johann Wolfgang Goethe-Universität Frankfurt, 60323 Frankfurt am Main (DE)
(72) Inventor: KRISHNAN, Jaya, 61476 Kronberg im Taunus (DE); YUAN, Ting, 65428 Rüsselsheim (DE)
(74) Representative: Schulz Junghans Patentanwälte PartGmbB
(86) International application number: PCT/EP2021/070690
(87) International publication number: WO 2022/018266

(56) References cited:
- WO-A1-2019/224812
- GULERIA RAKESHWAR S. ET AL: "Retinoic acid receptor-mediated signaling protects cardiomyocytes from hyperglycemia induced apoptosis: Role of the renin-angiotensin system", JOURNAL OF CELLULAR PHYSIOLOGY, vol. 226, no. 5, 13 October 2010 (2010-10-13), US, pages 1292 - 1307, XP055813782, ISSN: 0021-9541, DOI: 10.1002/jcp.22457
- IYER L M ET AL: "B-catenin/TCF7L2 signaling orchestrates initiation of pathological hypertrophic cardiac remodeling by inducing chromatin modifications", vol. 38, no. Issue suppl_1, 29 August 2017 (2017-08-29), pages 937, XP055813809, Retrieved from the Internet <URL:https://academic.oup.com/eurheartj/article-pdf/38/suppl_1/ehx504.P4479/19629019/ehx504.P4479.pdf>
- YE BO ET AL: "Opposing roles of TCF7/LEF1 and TCF7L2 in cyclin D2 and Bmp4 expression and cardiomyocyte cell cycle control during late heart development", LABORATORY INVESTIGATION, NATURE PUBLISHING GROUP, THE UNITED STATES AND CANADIAN ACADEMY OF PATHOLOGY, INC, vol. 99, no. 6, 18 February 2019 (2019-02-18), pages 807 - 818, XP036846900, ISSN: 0023-6837, [retrieved on 20190218], DOI: 10.1038/S41374-019-0204-2
- PONNUSAMY MURUGAVEL ET AL: "Understanding cardiomyocyte proliferation: an insight into cell cycle activity", CMLS CELLULAR AND MOLECULAR LIFE SCIENCES, BIRKHAUSER VERLAG, HEIDELBERG, DE, vol. 74, no. 6, 30 September 2016 (2016-09-30), pages 1019 - 1034, XP036157671, ISSN: 1420-682X, [retrieved on 20160930], DOI: 10.1007/S00018-016-2375-Y
- WERNER JOHN H. ET AL: "Molecular discoveries and treatment strategies by direct reprogramming in cardiac regeneration", TRANSLATIONAL RESEARCH, vol. 203, 31 July 2018 (2018-07-31), NL, pages 73 - 87, XP055813831, ISSN: 1931-5244, DOI: 10.1016/j.trsl.2018.07.012
- RECAMONDE-MENDOZA MARIANA ET AL: "Systems biology approach identifies key regulators and the interplay between miRNAs and transcription factors for pathological cardiac hypertrophy", GENE., vol. 698, 5 March 2019 (2019-03-05), NL, pages 157 - 169, XP055813822, ISSN: 0378-1119, DOI: 10.1016/j.gene.2019.02.056
- DAL-PRA SOPHIE ET AL: "Induced cardiomyocyte maturation: Cardiac transcription factors are necessary but not sufficient", vol. 14, no. 10, 17 October 2019 (2019-10-17), pages e0223842, XP055813821, Retrieved from the Internet <URL:https://storage.googleapis.com/plos-corpus-prod/10.1371/journal.pone.0223842/1/pone.0223842.pdf?X-Goog-Algorithm=GOOG4-RSA-SHA256&X-Goog-Credential=wombat-sa@plos-prod.iam.gserviceaccount.com/20210615/auto/storage/goog4_request&X-Goog-Date=20210615T082310Z&X-Goog-Expires=86400&X-Goog-SignedHeaders=h> DOI: 10.1371/journal.pone.0223842
- ZHU SEN ET AL: "Loss of myocardial retinoic acid receptor [alpha] induces diastolic dysfunction by promoting intracellular oxidative stress and calcium mishandling in adult mice", JOURNAL OF MOLECULAR AND CELLULAR CARDIOLOGY., vol. 99, 15 August 2016 (2016-08-15), GB, pages 100 - 112, XP055813780, ISSN: 0022-2828, DOI: 10.1016/j.yjmcc.2016.08.009

## Description

The present invention relates to a combination of inhibitory nucleic acid agents directed at a combination of transcription factors for treatment of heart failure. The combination of inhibited transcription factors comprises one of the groups selected from RARa, TCF7L2, E2F6 and LEF1, or RARa, TCF7L2, E2F7 and NFYb. The combinatorial inhibition of these transcription factors is shown to increase cardiomyocyte proliferation.

### Background of the Invention

Cardiovascular diseases are currently the leading causes of death in the world. Particularly heart failure, that affects 64 million people worldwide, is the major cause of cardiovascular morbidity and mortality. Massive cardiomyocyte death occurs during acute myocardial infarction (AMI), but the remaining cardiomyocytes have very limited regenerative capacity to restore the damaged tissue, leading to cardiac fibrosis and heart failure. Therefore, cardiac regeneration therapy may be the next best solution to cure cardiac pathologies, including heart failure, ischemia/reperfusion and myocardial infarction.

WO 2019 224 812 A1 describes the treatment of cardiovascular diseases such as heart failure with inhibitors of the transcription factors ATF3 and JDP2. Guleria et al. (Journal of Cellular Physiology, vol. 226, no. 5 (2010)) examines the role of the renin-angiotensin system in diabetes mellitus. Iyer et al. (European Heart Journal, vol. 38, no. Issue suppl_1 (2017)) investigates the consequences of activating the Wnt pathway in an adult heart via the transcription factor TCF7L2.

Hence, understanding the mechanisms that drive cardiomyocyte proliferation is essential to establish novel therapeutic avenues for heart failure.

Based on the above-mentioned state of the art, the objective of the present invention is to provide means and methods to treat heart failure. This objective is attained by the subjectmatter of the independent claims of the present specification.

### Summary of the Invention

A first aspect of the invention relates to a combination of nucleic acid agents for use in treatment or prevention of heart disease. Each of said nucleic acid agents is capable of specifically inhibiting the expression of a target protein. The combination of nucleic acid agents is capable of inhibiting the expression of a combination of target proteins (transcription factors) within a cell.

The combination of target proteins is
a. RARa, and
b. TCF7L2, and
c. E2F6 and LEF1, or
   E2F7 and NFYb.

A second aspect of the invention relates to an expression construct, or a plurality of expression constructs, encoding, under control of a promoter operable in a human myocardial cell, a combination of nucleic acid agents for use in treatment or prevention of heart disease. Each of the nucleic acid agents is capable of specifically inhibiting the expression of a target protein, thereby inhibiting the expression of a combination of target proteins (transcription factors). The combination of target proteins is
a. RARa, and
b. TCF7L2, and
c. E2F6 and LEF1, or
   E2F7 and NFYb.

A third aspect of the invention relates to a pharmaceutical composition for use in treatment or prevention of heart disease comprising the combination of nucleic acid agents according to the first aspect, or the plurality of expression constructs according to the second aspect.

In another embodiment, the present invention relates a pharmaceutical composition comprising at least one of the compounds of the present invention or a pharmaceutically acceptable salt thereof and at least one pharmaceutically acceptable carrier, diluent or excipient.

### Detailed Description

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The terms *capable of forming a hybrid* or *hybridizing sequence* in the context of the present specification relate to sequences that under the conditions existing within the cytosol of a mammalian cell, are able to bind selectively to their target sequence. Such hybridizing sequences may be contiguously reverse-complimentary to the target sequence, or may comprise gaps, mismatches or additional non-matching nucleotides. The minimal length for a sequence to be capable of forming a hybrid depends on its composition, with C or G nucleotides contributing more to the energy of binding than A or T/U nucleotides, and on the backbone chemistry.

The term *Nucleotides* in the context of the present specification relates to nucleic acid or nucleic acid analogue building blocks, oligomers of which are capable of forming selective hybrids with RNA or DNA oligomers on the basis of base pairing. The term *nucleotides* in this context includes the classic ribonucleotide building blocks adenosine, guanosine, uridine (and ribosylthymine), cytidine, the classic deoxyribonucleotides deoxyadenosine, deoxyguanosine, thymidine, deoxyuridine and deoxycytidine. It further includes analogues of nucleic acids such as phosphotioates, 2'O-methylphosphothioates, *peptide nucleic acids* (PNA; N-(2-aminoethyl)-glycine units linked by peptide linkage, with the nucleobase attached to the alpha-carbon of the glycine) or *locked nucleic acids* (LNA; 2'O, 4'C methylene bridged RNA building blocks). Wherever reference is made herein to a *hybridizing sequence,* such hybridizing sequence may be composed of any of the above nucleotides, or mixtures thereof.

The term *siRNA* (small/short interfering RNA) in the context of the present specification relates to an RNA molecule capable of interfering with the expression (in other words: inhibiting or preventing the expression) of a gene comprising a nucleic acid sequence complementary or hybridizing to the sequence of the siRNA in a process termed RNA interference. The term *siRNA* is meant to encompass both single stranded siRNA and double stranded siRNA. siRNA is usually characterized by a length of 17-24 nucleotides. Double stranded siRNA can be derived from longer double stranded RNA molecules (dsRNA). According to prevailing theory, the longer dsRNA is cleaved by an endo-ribonuclease (called Dicer) to form double stranded siRNA. In a nucleoprotein complex (called RISC), the double stranded siRNA is unwound to form single stranded siRNA. RNA interference often works via binding of an siRNA molecule to the mRNA molecule having a complementary sequence, resulting in degradation of the mRNA. RNA interference is also possible by binding of an siRNA molecule to an intronic sequence of a pre-mRNA (an immature, non-spliced mRNA) within the nucleus of a cell, resulting in degradation of the pre-mRNA.

The term *shRNA* (small hairpin RNA) in the context of the present specification relates to an artificial RNA molecule with a tight hairpin turn that can be used to silence target gene expression via RNA interference (RNAi).

The term *sgRNA* (single guide RNA) in the context of the present specification relates to an RNA molecule capable of sequence-specific repression of gene expression via the CRISPR (clustered regularly interspaced short palindromic repeats) mechanism.

The term *miRNA* (microRNA) in the context of the present specification relates to a small noncoding RNA molecule (containing about 22 nucleotides) that functions in RNA silencing and post-transcriptional regulation of gene expression.

The term *antisense oligonucleotide* in the context of the present specification relates to an oligonucleotide having a sequence substantially complimentary to, and capable of hybridizing to, an RNA. Antisense action on such RNA will lead to modulation, particular inhibition or suppression of the RNA's biological effect. If the RNA is an mRNA, expression of the resulting gene product is inhibited or suppressed. Antisense oligonucleotides can consist of DNA, RNA, nucleotide analogues and/or mixtures thereof. The skilled person is aware of a variety of commercial and non-commercial sources for computation of a theoretically optimal antisense sequence to a given target. Optimization can be performed both in terms of nucleobase sequence and in terms of backbone (ribo, deoxyribo, analogue) composition. Many sources exist for delivery of the actual physical oligonucleotide, which generally is synthesized by solid state synthesis. Examples of antisense oligonucleotides of the present invention are antisense oligomers made of DNA, DNA having phosphorothioate modified linkages in their backbone, ribonucleotide oligomers, RNA comprising bridged or locked nucleotides, particularly wherein the ribose ring is connected by a methylene bridge between the 2'-O and 4'-C atoms, RNA having phosphorothioate modified linkages in their backbone or any mixture of deoxyribonucleotide and ribonucleotide bases as an oligomer.

In certain embodiments, the antisense oligonucleotide of the invention comprises analogues of nucleic acids such as phosphotioates, 2'O-methylphosphothioates, peptide nucleic acids (PNA; N-(2-aminoethyl) -glycine units linked by peptide linkage, with the nucleobase attached to the alpha-carbon of the glycine) or locked nucleic acids (LNA; 2'O, 4'C methylene bridged RNA building blocks). The antisense sequence may be composed partially of any of the above analogues of nucleic acids, with the rest of the nucleotides being "native" ribonucleotides occurring in nature, or may be mixtures of different analogues, or may be entirely composed of one kind of analogue.

The term *nucleic acid expression vector* in the context of the present specification relates to a plasmid or a viral genome, which is used to transfect (in case of a plasmid) or transduce (in case of a viral genome) a target cell with a certain gene of interest. The gene of interest is under control of a promoter sequence and the promoter sequence is operational inside the target cell, thus, the gene of interest is transcribed either constitutively or in response to a stimulus or dependent on the cell's status. In certain embodiments, the viral genome is packaged into a capsid to become a viral vector, which is able to transduce the target cell.

Sequences similar or homologous (e.g., at least about 70% sequence identity) to the sequences disclosed herein are also part of the invention. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

In the context of the present specification, the terms *sequence identity* and *percentage of sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless stated otherwise, sequence identity values provided herein refer to the value obtained using the BLAST suite of programs (Altschul et al., J. Mol. Biol. 215:403-410 (1990)) using the above identified default parameters for protein and nucleic acid comparison, respectively.

Reference to identical sequences without specification of a percentage value implies 100% identical sequences (i.e. the same sequence).

In the context of the present specification, the term *hybridizing sequence* encompasses a polynucleotide sequence comprising or essentially consisting of RNA (ribonucleotides), DNA (deoxyribonucleotides), phosphothioate deoxyribonucleotides, 2'-O-methyl-modified phosphothioate ribonucleotides, LNA and/or PNA nucleotide analogues.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

The term RARa in the context of the present specification relates to retinoic acid receptor alpha. Homo sapiens RARa: Gene ID: 5914; Rattus norvegicus RARa: Gene ID: 24705; Mus musculus RARa: Gene ID: 19401.

The term TCF7L2 in the context of the present specification relates to transcription factor 7 like 2.

Homo sapiens TCF7L2: Gene ID: 6934; Rattus norvegicus TCF7L2: Gene ID: 679869; Mus musculus TCF7L2: Gene ID: 21416.

The term E2F6 in the context of the present specification relates to E2F transcription factor 6. Homo sapiens E2F6: Gene ID: 1876; Rattus norvegicus E2F6: Gene ID: 313978; Mus musculus E2F6: Gene ID: 50496.

The term LEF1 in the context of the present specification relates to lymphoid enhancer binding factor 1.

Homo sapiens LEF1: Gene ID: 51176; Rattus norvegicus LEF1: Gene ID: 16842; Mus musculus LEF1: Gene ID: 161452.

The term E2F7 in the context of the present specification relates to E2F transcription factor 7. Homo sapiens E2F7: Gene ID: 144455; Rattus norvegicus E2F7: Gene ID: 52679; Mus musculus E2F7: Gene ID: 314818.

The term NFYb in the context of the present specification relates to nuclear transcription factor Y subunit beta.

Homo sapiens NFYb: Gene ID: 4801; Rattus norvegicus NFYb: Gene ID: 18045; Mus musculus NFYb: Gene ID: 25336.

A first aspect of the invention relates to a combination of nucleic acid agents for use in treatment or prevention of heart disease. Each of said nucleic acid agents is capable of specifically inhibiting the expression of a target protein. The combination of nucleic acid agents is capable of inhibiting the expression of a combination of target proteins (transcription factors) within a cell. The combination of target proteins is
a. RARa, and
b. TCF7L2, and
c. E2F6 and LEF1, or
   E2F7 and NFYb.

In certain embodiments, the combination of target proteins is
a. RARa, and
b. TCF7L2, and
c. E2F6, and
d. LEF1.

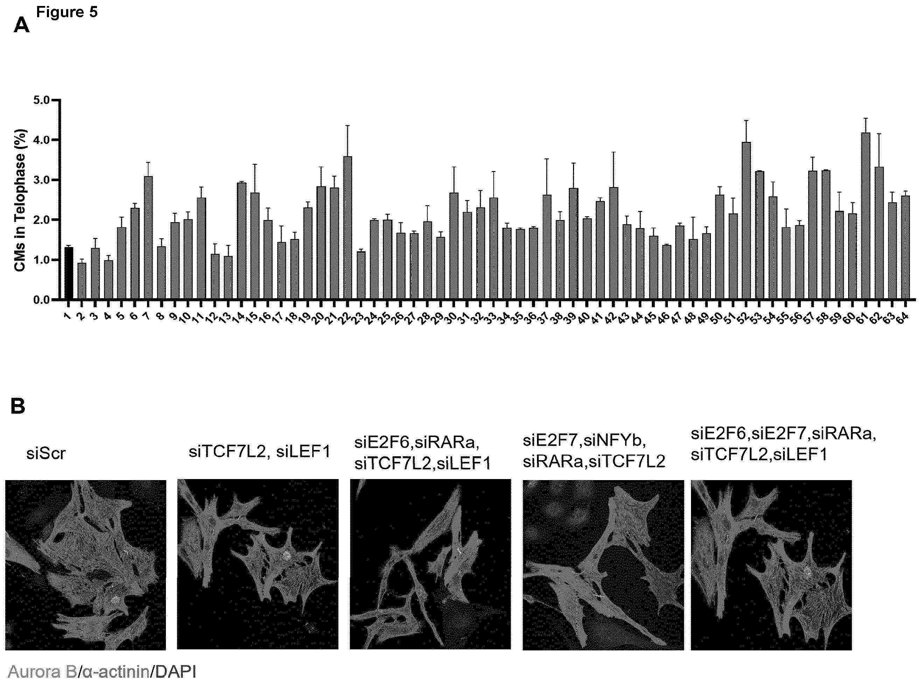

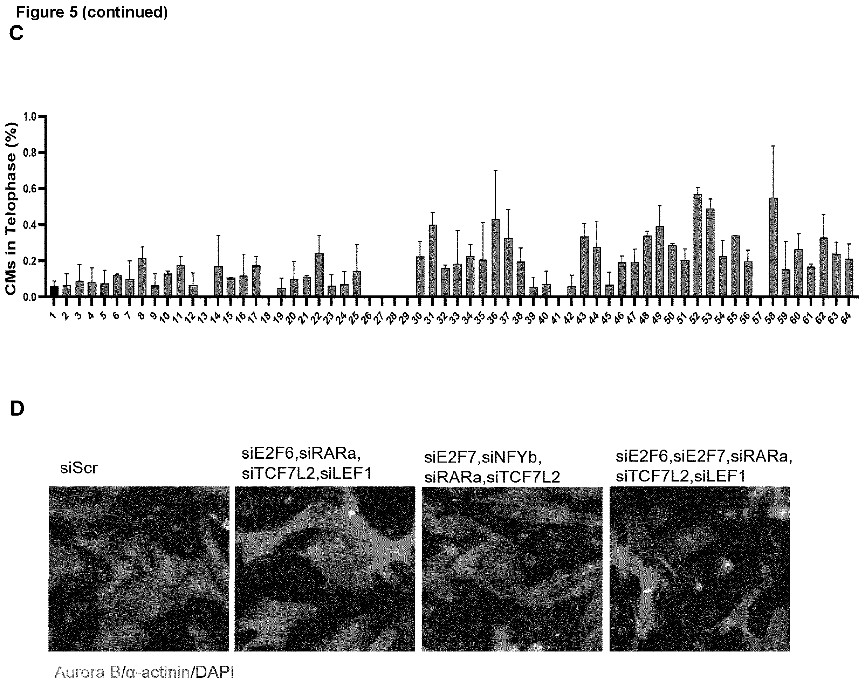

The combinations of nucleic acid agents were tested in P7 rat cardiomyocytes, when cell division had ceased (Fig. 5). This situation resembles the treatment situation in heart failure best, because also in the heart failure treatment situation, cell division of cardiomyocytes has ceased. The inventors found that the combination of RARa, TCF7L2, E2F6, and LEF1 induces the highest cell division rate (Fig. 5), measured as percentage of cardiomyocytes in Telophase.

In certain embodiments, each nucleic acid agent comprises a hybridizing sequence of nucleotides, wherein the hybridizing sequence of nucleotides is capable of forming a hybrid with its respective mRNA encoding the target protein. In certain embodiments, each nucleic acid agent essentially consists of a hybridizing sequence of nucleotides, wherein the hybridizing sequence of nucleotides is capable of forming a hybrid with its respective mRNA encoding the target protein.

In certain embodiments, each nucleic acid agent is selected independently from an antisense oligonucleotide, an siRNA, an shRNA, an sgRNA and an miRNA. In certain embodiments, each nucleic acid agent is an siRNA.

In certain embodiments, the sense strand is modified to prevent interaction with RISC and favor antisense strand uptake. In certain embodiments, the antisense strand seed region is modified to destabilize off-target activity and enhance target specificity.

In certain embodiments, one or two of said nucleic acid agents or each nucleic acid agent comprises or consists of nucleoside analogues.

In certain embodiments, each nucleic acid agent comprises a (different) sequence selected from the sequences SEQ ID NO 165-188. In certain embodiments, each nucleic acid agent essentially consists of a (different) sequence selected from the sequences SEQ ID NO 165-188.

In certain embodiments, one of said nucleic acid agents comprises one or several locked nucleic acid (LNA) and/or peptide nucleic acid (PNA) moieties. In certain embodiments, one of said nucleic acid agents essentially consists of one or several locked nucleic acid (LNA) and/or peptide nucleic acid (PNA) moieties.

In certain embodiments, several or all of said nucleic acid agents comprises one or several locked nucleic acid (LNA) and/or peptide nucleic acid (PNA) moieties. In certain embodiments, several or all of said nucleic acid agents essentially consists of one or several locked nucleic acid (LNA) and/or peptide nucleic acid (PNA) moieties.

In certain embodiments, the nucleic acid agents essentially consist of locked nucleic acid (LNA) moieties.

In certain embodiments, one of the nucleic acid agents comprises one or several thiophosphate linkages connecting adjacent (deoxy)ribonucleoside moieties or ribonucleotide analogue moieties. In certain embodiments, several or all of the nucleic acid agents comprise one or several thiophosphate linkages connecting adjacent (deoxy)ribonucleoside moieties or ribonucleotide analogue moieties.

In certain embodiments, one of said nucleic acid agents comprises a LNA moiety connected to an adjacent ribonucleoside or ribonucleotide analogue moiety by a thiophosphate bond. In certain embodiments, one of said nucleic acid agents comprises 2, 3 or 4 LNA moieties connected by thiophosphate bonds on either end of the nucleic acid agent.

In certain embodiments, several or all of the nucleic acid agents comprises a LNA moiety connected to an adjacent ribonucleoside or ribonucleotide analogue moiety by a thiophosphate bond. In certain embodiments, several or all of the nucleic acid agents comprises 2, 3 or 4 LNA moieties connected by thiophosphate bonds on either end of the nucleic acid agent.

In certain embodiments, one of said nucleic acid agents is expressed intracellularly from an exogenous (transgene) expression construct comprising a sequence encoding a hybridizing sequence of nucleotides under control of a promoter operable in a human myocardial cell.

In certain embodiments, several or all of said nucleic acid agents is expressed intracellularly from an exogenous (transgene) expression construct comprising a sequence encoding a hybridizing sequence of nucleotides under control of a promoter operable in a human myocardial cell.

A second aspect of the invention relates to an expression construct, or a plurality of expression constructs, encoding, under control of a promoter operable in a human myocardial cell, a combination of nucleic acid agents for use in treatment or prevention of heart disease. Each of the nucleic acid agents is capable of specifically inhibiting the expression of a target protein, thereby inhibiting the expression of a combination of target proteins (transcription factors). The combination of target proteins is
a. RARa, and
b. TCF7L2, and
c. E2F6 and LEF1, or
   E2F7 and NFYb.

In certain embodiments, the combination of target proteins is
a. RARa, and
b. TCF7L2, and
c. E2F6, and
d. LEF1.

In certain embodiments, each and all of said expression constructs are comprised within a single nucleic acid molecule.

In certain embodiments, the single nucleic acid molecule comprising all of the expression constructs is a polycistronic construct, which has several open-reading frames, one for each of the combination of nucleic acid agents of the invention.

In certain embodiments, said nucleic acid agents or said expression construct are/is bound to a nanoparticle or encapsulated by a particulate structure selected from a virus and a liposome.

A liposome is a spherical vesicle having at least one lipid bilayer. Liposomes can be prepared by disrupting biological membranes (e.g. by sonication). The major types of liposomes are the multilamellar vesicle (MLV, with several lamellar phase lipid bilayers), the small unilamellar liposome vesicle (SUV, with one lipid bilayer), the large unilamellar vesicle (LUV), and the cochleate vesicle.

A nanoparticle is a particle of matter that is between 1 and 100 nanometres (nm) in diameter. In certain embodiments, the nanoparticle is composed of a material selected from silver, gold, hydroxyapatite, clay, titanium dioxide, silicon dioxide, zirconium dioxide, carbon, diamond, aluminium oxide and ytterbium trifluoride.

In certain embodiments, the virus is selected from a retrovirus, a lentivirus, an adenovirus, and an adeno-associated virus, or a hybrid of the afore-mentioned. In certain embodiments, the virus selectively transduces cardiomyocytes.

In certain embodiments, the nanoparticle, or the particulate structure comprise a ligand facilitating delivery to or entry of the nanoparticle or the particulate structure into a cardiomyocyte. In certain embodiments, the ligand is selected from an anti-CD71 Fab' fragment and an anti-cTnl Fab' fragment. These ligands function by targeting a cardiomyocyte-specific cell surface receptor for targeted delivery.

In certain embodiments, the promoter allows for heart-specific expression of said nucleic acid agents. In certain embodiments, the promoter is a heart-tissue-specific RNA Polymerase 2 promoter. In certain embodiments, the promoter is selected from Myosin Light Chain 2v (MLC2v) promoter, cardiac Troponin T (cTnT) promoter, Myosin Heavy Chain alpha (MHCa) promoter, and Titin (Ttn) promoter.

In certain embodiments, the heart disease is characterized by a loss of cardiomyocytes. In certain embodiments, the heart disease is heart failure characterized by a loss of cardiomyocytes. In certain embodiments, the heart failure is a result of myocardial infarction, stenosis, congenital disease, inflammation and/or sepsis.

In certain embodiments, the heart disease is characterized by hypoxia of cardiomyocytes.

Heart failure (HF) occurs when the heart is unable to pump sufficiently to maintain blood flow to meet the body's needs and leads to death if untreated. HF is the culmination of disease progression in indications including coronary artery disease, ischemic heart disease, aortic stenosis, hypertension and metabolic disease. Heart failure may be caused by a loss of cardiomyocytes. Cardiomyocytes are often lost due to insufficient supply with oxygen (hypoxia). The remaining cardiomyocytes are triggered to divide and replace the lost cardiomyocytes using the combination of nucleic acid agents of the present invention.

A third aspect of the invention relates to a pharmaceutical composition for use in treatment or prevention of heart disease comprising the combination of nucleic acid agents according to the first aspect, or the plurality of expression constructs according to the second aspect.

In certain embodiments, the pharmaceutical composition is administered to the heart.

### Medical treatment, Dosage Forms and Salts

Similarly, a dosage form for the prevention or treatment of heart disease is provided, comprising an antisense molecule according to any of the above aspects or embodiments of the invention.

The skilled person is aware that any specifically mentioned drug may be present as a pharmaceutically acceptable salt of said drug. Pharmaceutically acceptable salts comprise the ionized drug and an oppositely charged counterion. Non-limiting examples of pharmaceutically acceptable anionic salt forms include acetate, benzoate, besylate, bitatrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminium, benzathine, calcium, ethylene diamine, lysine, magnesium, meglumine, potassium, procaine, sodium, tromethamine and zinc.

Dosage forms may be for enteral administration, such as nasal, buccal, rectal, transdermal or oral administration, or as an inhalation form or suppository. Alternatively, parenteral administration may be used, such as subcutaneous, intravenous, intrahepatic or intramuscular injection forms. Optionally, a pharmaceutically acceptable carrier and/or excipient may be present.

### Pharmaceutical Compositions and Administration

Another aspect of the invention relates to a pharmaceutical composition comprising a compound of the present invention, or a pharmaceutically acceptable salt thereof, and a pharmaceutically acceptable carrier. In further embodiments, the composition comprises at least two pharmaceutically acceptable carriers, such as those described herein.

In certain embodiments of the invention, the compound of the present invention is typically formulated into pharmaceutical dosage forms to provide an easily controllable dosage of the drug and to give the patient an elegant and easily handleable product.

The pharmaceutical composition can be formulated for oral administration, parenteral administration, or rectal administration. In addition, the pharmaceutical compositions of the present invention can be made up in a solid form (including without limitation capsules, tablets, pills, granules, powders or suppositories), or in a liquid form (including without limitation solutions, suspensions or emulsions).

The dosage regimen for the compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

In certain embodiments, the pharmaceutical composition or combination of the present invention can be in unit dosage of about 1-1000 mg of active ingredient(s) for a subject of about 50-70 kg. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

The invention is further illustrated by the following examples and figures, from which further embodiments and advantages can be drawn. These examples are meant to illustrate the invention but not to limit its scope.

### Description of the Figures

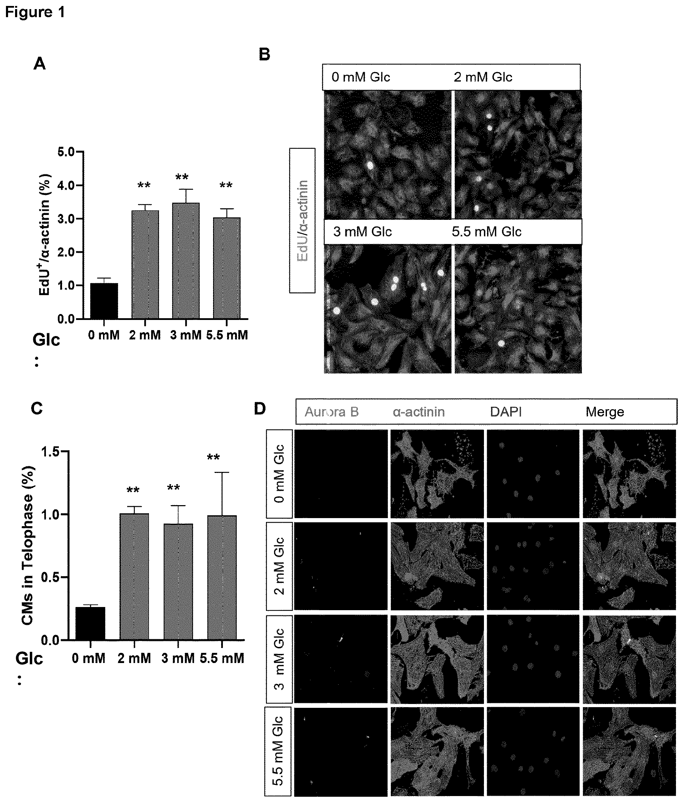

Figure 1 Proliferation on neonatal rat cardiomyocytes *in vitro.* Neonatal rat cardiomyocytes were treated with glucose (0 mM, 2 mM, 3mM and 5.5 mM) for 2 days, then the cardiomyocyte proliferation were analyzed by 5-ethynyl-2'-deoxyuridine (EdU) incorporation and Aurora B staining. (A) Representative immunofluorescence images of EdU on cardiomyocytes. EdU labels proliferating cells (green); cardiomyocyte-specific α-actinin (red) and DAPI (Blue). (B) Quantification of percentages of EdU⁺ cardiomyocytes. (C) Representative immunofluorescence images of Aurora B on cardiomyocytes. Aurora B labels proliferating cells (green); cardiomyocyte-specific α-actinin (red) and DAPI (Blue). (D) Quantification of percentages of Aurora B⁺ cardiomyocytes. Data are presented as mean ± SEM. *P<0.05, **P<0.01. Statistical analysis was performed by the 2-tailed Student's t test.

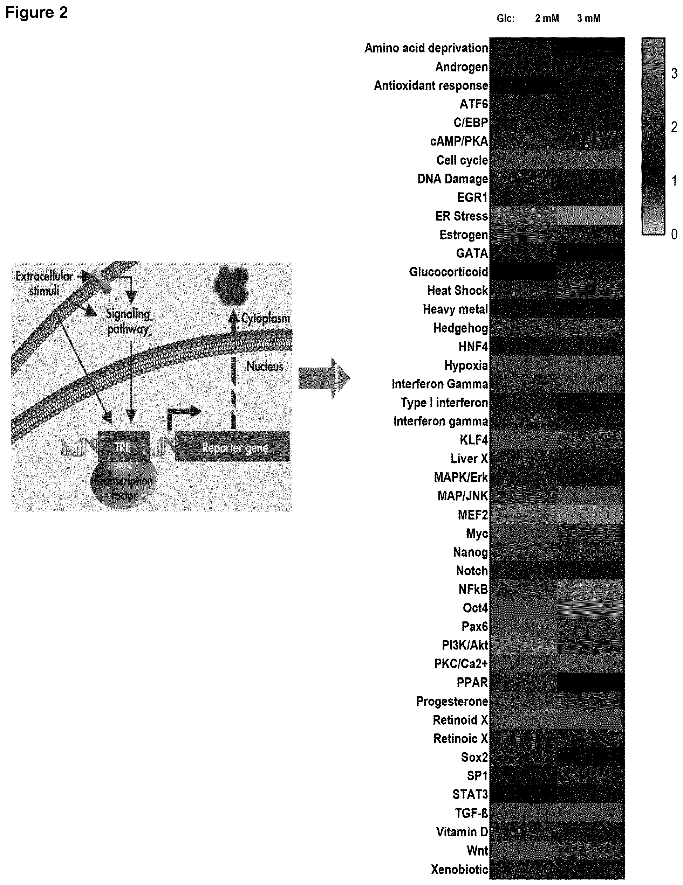

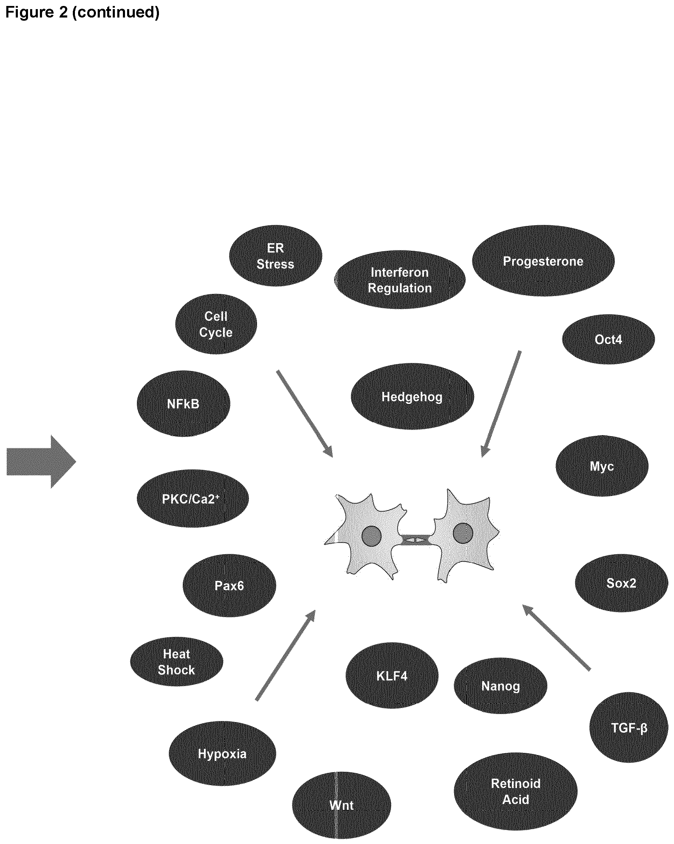

Figure 2 Cignal Reporter Assay. Neonatal rat cardiomyocytes were transfected with a mixture of transcription factor-responsive firefly luciferase reporter and a constitutively expressing Renilla contruct, then the next day cells were treated with glucose (0 mM, 2 mM and 3mM) for 2 days. Luciferase activity was assessed using the Dual-Glo Luciferase Assay System. Heatmap shows that 18 signaling pathways were downregulated, as indicated by reporter activity. Among the 18 signaling pathways, the inventors have selected 34 transcription factors as the candidates for the following experiments.

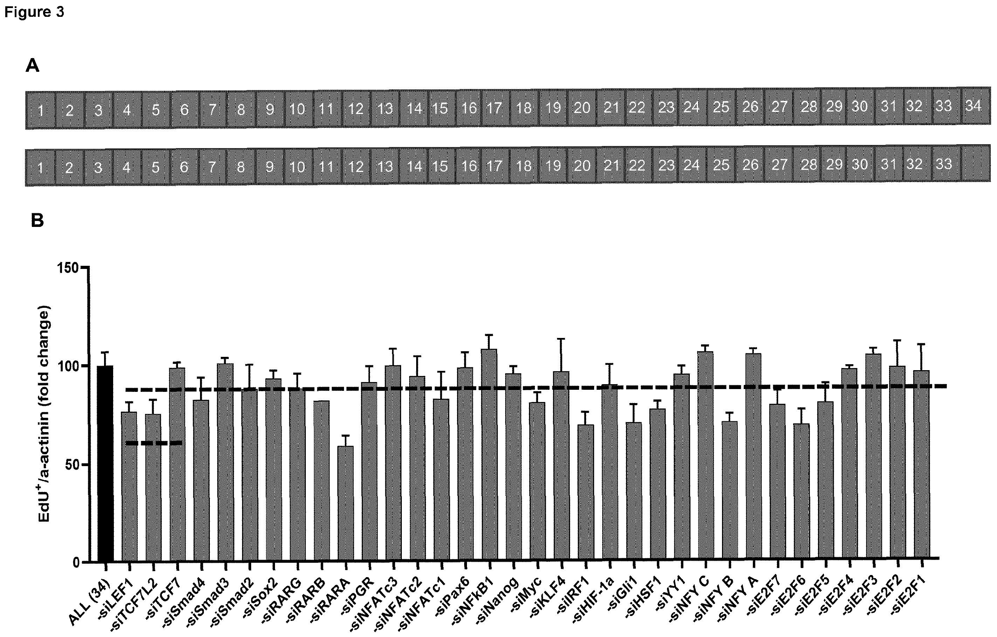

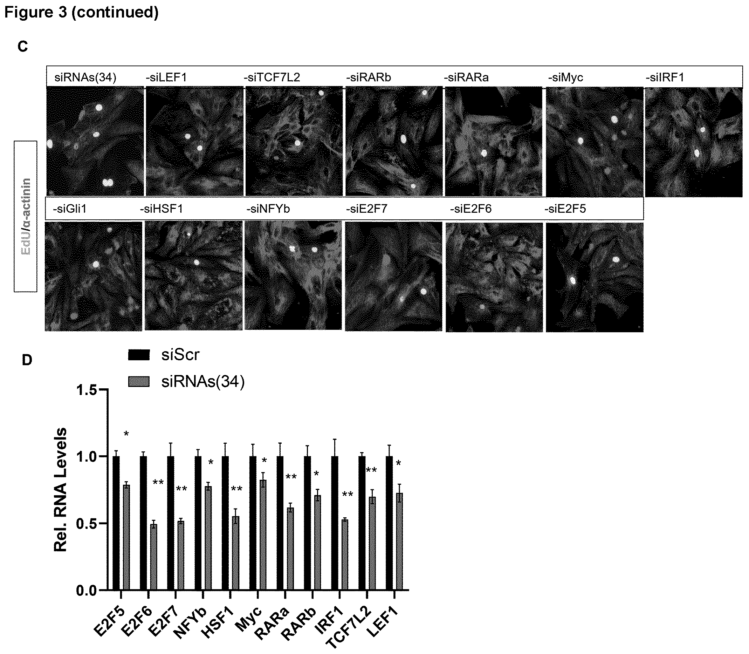

Figure 3 Narrowing down the candidate transcription factors by EdU screening. The inventors selected 34 genes as candidates for factors that increase cardiomyocyte proliferation, and the selected transcription factors were knockdown by ON-TARGETplus SMARTpool siRNA. Neonatal rat cardiomyocytes were transfected of removal of individual factors from the pool of 34 siRNAs, while the cells were transfected with 34 siRNAs together as control (A). After 3 days transfection, the DNA synthesis (EdU) was analyzed by double staining for EdU (green) and α-actinin (red) (B, C). (D) The gene expression levels were analyzed by qRT-PCR. Data are expressed as mean ± SEM. *P<0.05, **P<0.01. Statistical analysis was performed by the 2-tailed Student's t test.

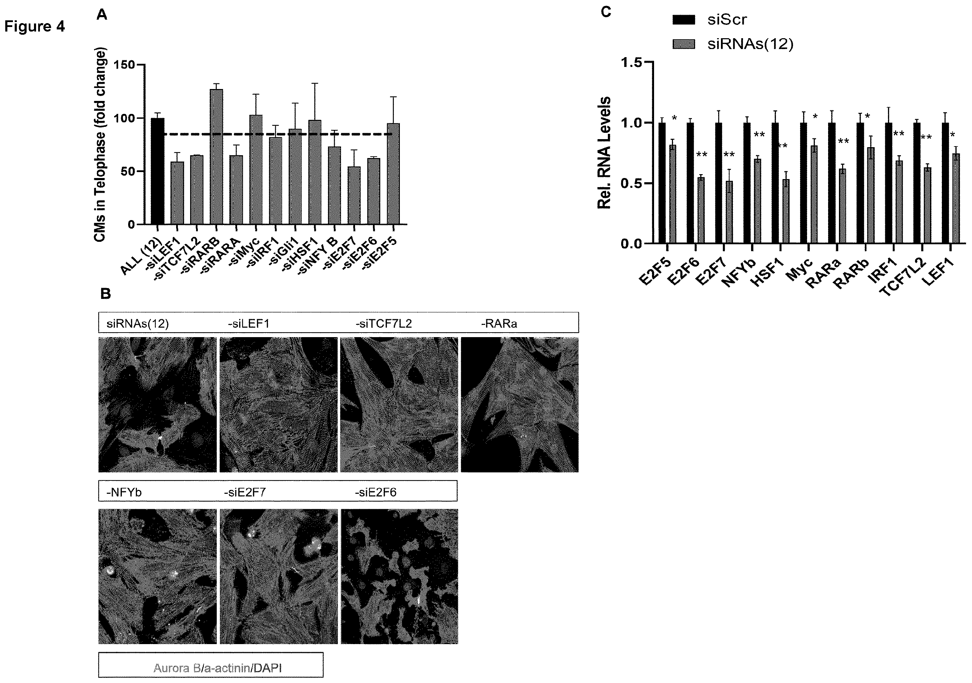

Figure 4 Narrowing down the candidate transcription factors by Aurora B. Based on the EdU screening, the inventors selected 12 genes which are necessary for DNA synthesis, then the inventors used Aurora B as another readout for cytokinesis. Neonatal rat cardiomyocytes were transfected of removal of individual factors from the pool of 12 siRNAs, while the cells were transfected with 12 siRNAs together as control. After 3 days transfection, cardiomyocyte proliferation was analyzed by triple staining for Aurora B (green), α-actinin (red) and DAPI (blue) (A, B). (C) The gene expression levels were analyzed by qRT-PCR. Data are expressed as mean ± SEM. *P<0.05, **P<0.01. Statistical analysis was performed by the 2-tailed Student's t test.
Figure 5 Cell proliferation on rat cardiomyocytes *in vitro*
Based on the EdU and Aurora B screening, the inventors selected 6 genes which are necessary for DNA synthesis and cytokinesis. To identify the best combinatorial factors, all combinatorial siRNAs were transfected in the 1-day-old (A, B) and 7-old (C, D) rat cardiomyocytes, and then the cardiomyocyte proliferation was analyzed by triple staining for Aurora B (green), α-actinin (red) and DAPI (blue). At the end, the inventors selected 2 best combinations which could increase cardiomyocyte proliferation.

| | |
|---|---|
| 1 | siScr |
| 2 | siE2F6 |
| 3 | siE2F7 |
| 4 | siNFYb |
| 5 | siRARa |
| 6 | siTCF7L2 |
| 7 | siLEF1 |
| 8 | siE2F6,siE2F7 |
| 9 | siE2F6,siNFYb |
| 10 | siE2F6,siRARa |
| 11 | siE2F6,siTCF7L2 |
| 12 | siE2F6,siLEF1 |
| 13 | siE2F7,siNFYb |
| 14 | siE2F7,siRARa |
| 15 | siE2F7,siTCF7L2 |
| 16 | siE2F7,siLEF1 |
| 17 | siNFYb,siRARa |
| 18 | siNFYb,siTCF7L2 |
| 19 | siNFYb,siLEF1 |
| 20 | siRARa,siTCF7L2 |
| 21 | siRARa,siLEF1 |
| 22 | siTCF7L2,siLEF1 |
| 23 | siE2F6,siE2F7,siNFYb |
| 24 | siE2F6,siE2F7,siRARa |
| 25 | siE2F6,siE2F7,siTCF7L2 |
| 26 | siE2F6,siE2F7,siLEF1 |
| 27 | siE2F6,siNFYb,siRARa |
| 28 | siE2F6,siNFYb,siTCF7L2 |
| 29 | siE2F6,siNFYb,siLEF1 |
| 30 | siE2F6,siRARa,siTCF7L2 |
| 31 | siE2F6,siRARa,siLEF1 |
| 32 | siE2F6,siTCF7L2,siLEF1 |
| 33 | siE2F7,siNFYb,siRARa |
| 34 | siE2F7,siNFYb,siTCF7L2 |
| 35 | siE2F7,siNFYb,siLEF1 |
| 36 | siE2F7,siRARa,siTCF7L2 |
| 37 | siE2F7,siRARa,siLEF1 |
| 38 | siE2F7,siTCF7L2,siLEF1 |
| 39 | siNFYb,siRARa,siTCF7L2 |
| 40 | siNFYb,siRARa,siLEF1 |
| 41 | siNFYb,siTCF7L2,siLEF1 |
| 42 | siRARa,siTCF7L2,siLEF1 |
| 43 | siE2F6,siE2F7,siNFYb,siRARa |
| 44 | siE2F6,siE2F7,siNFYb,siTCF7L2 |
| 45 | siE2F6,siE2F7,siNFYb,siLEF1 |
| 46 | siE2F6,siE2F7,siRARa,siTCF7L2 |
| 47 | siE2F6,siE2F7,siRARa,siLEF1 |
| 48 | siE2F6,siE2F7,siTCF7L2,siLEF1 |
| 49 | siE2F6,siNFYb,siRARa,siTCF7L2 |
| 50 | siE2F6,siE2F7,siTCF7L2,siLEF1 |
| 51 | siE2F6,siNFYb,siRARa,siLEF1 |
| 52 | siE2F6,siRARa,siTCF7L2,siLEF1 |
| 53 | siE2F7,siNFYb,siRARa,siTCF7L2 |
| 54 | siE2F7,siNFYb,siRARa,siLEF1 |
| 55 | siE2F7,siRARa,siTCF7L2,siLEF1 |
| 56 | siE2F7,siNFYb,siTCF7L2,siLEF1 |
| 57 | siNFYb,siRARa,siTCF7L2,siLEF1 |
| 58 | siE2F6,siE2F7,siNFYb,siRARa,siTCF7L2 |
| 59 | siE2F6,siE2F7,siNFYb,siRARa,siLEF1 |
| 60 | siE2F6,siE2F7,siNFYb,siTCF7L2,siLEF1 |
| 61 | siE2F6,siE2F7,siRARa,siTCF7L2,siLEF1 |
| 62 | siE2F6,siNFYb,siRARa,siTCF7L2,siLEF1 |
| 63 | siE2F7,siNFYb,siRARa,siTCF7L2,siLEF1 |
| 64 | siE2F6,siE2F7,siNFYb,siRARa,siTCF7L2,siLEF1 |

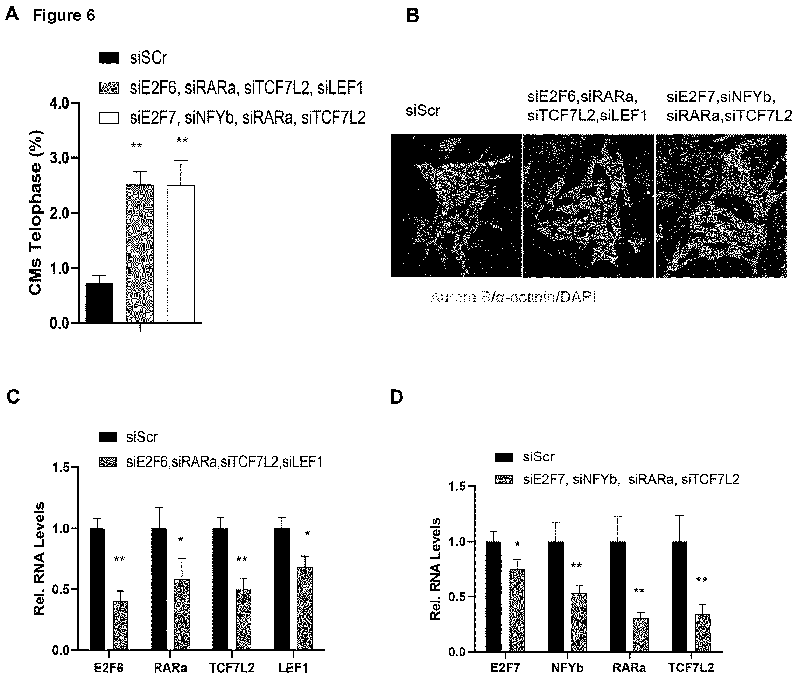

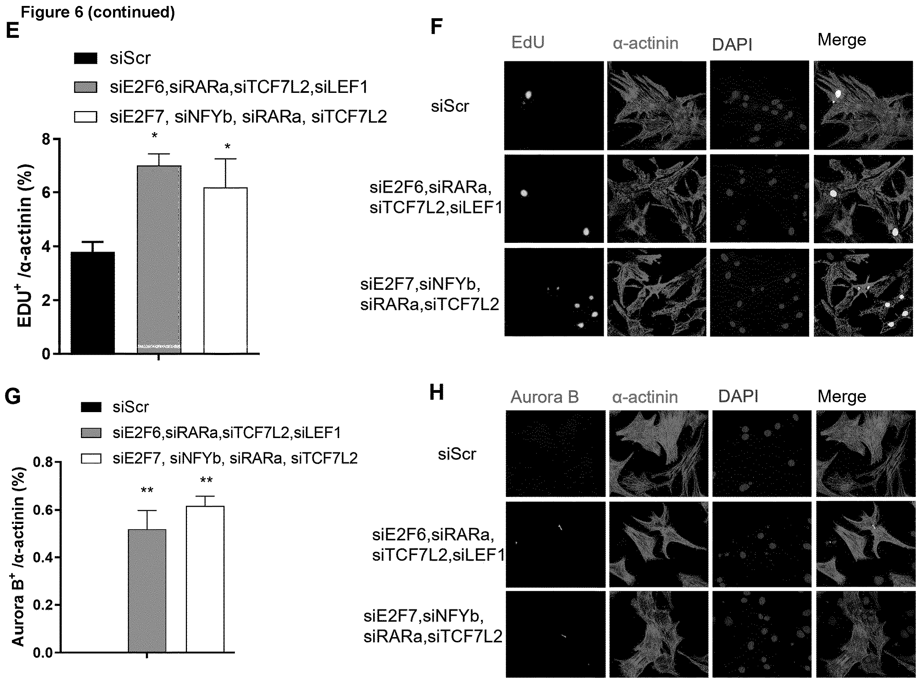

Figure 6 Rat cardiomyocyte proliferation upon E2F6/RARa/TCF7L2/LEF1 or E2F7/NFYb/RARa/TCF7L2 knockdown
Rat cardiomyocytes (P1) were transfected with siE2F6, siRARa, siTCF7L2 and siLEF1; siE2F7, siNFYb, siRARa and siTCF7L2 or control scramble siRNA (siScr) for 3 days and cardiomyocyte proliferation was analyzed and quantified by triple staining for Aurora B (green), α-actinin (red), and DAPI (blue) (A, B). (C, D) Respective gene expression levels were analyzed by qRT-PCR. (E, F) P7 rat cardiomyocytes were transfected with siE2F6, siRARa, siTCF7L2 and siLEF1; siE2F7, siNFYb, siRARa and siTCF7L2 or control scramble siRNA (siScr) for 3 days, then cardiomyocyte proliferation was analyzed and quantified by triple staining for EdU (green), α-actinin (red), and DAPI (blue). (G, H) P7 rat cardiomyocytes were transfected with siE2F6, siRARa, siTCF7L2 and siLEF1; siE2F7, siNFYb, siRARa and siTCF7L2 or control scramble siRNA (siScr) for 3 days, then cardiomyocyte proliferation was analyzed and quantified by triple staining for Aurora B (green), α-actinin (red), and DAPI (blue). Data are expressed as mean ± SEM. *P<0.05, **P<0.01. Statistical analysis was performed by the 2-tailed Student's t test.

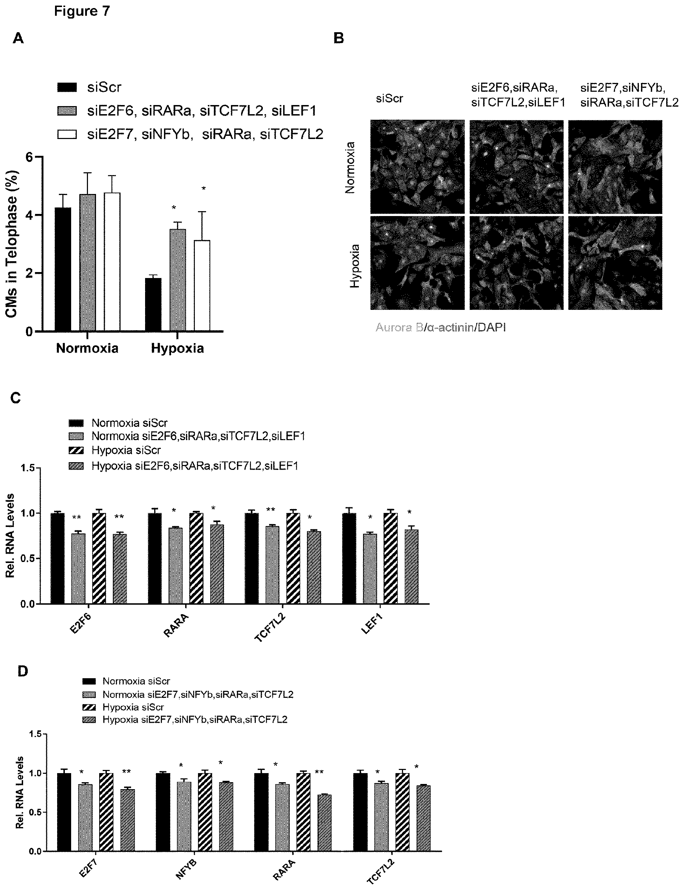

Figure 7 Human iPS-CM proliferation upon E2F6/RARa/TCF7L2/LEF1 or E2F7/NFYb/RARa/TCF7L2 knockdown
Human iPSC-CMs were transfected with siE2F6, siRARa, siTCF7L2 and siLEF1; siE2F7, siNFYb, siRARa and siTCF7L2 or control scramble siRNA (siScr) , and then the cells were exposed to normoxia (20% O₂) or hypoxia (3% O₂) for 2 days as an *in vitro* model for myocardial infarction (MI). Cardiomyocytes proliferation was analyzed and quantified by triple staining for Aurora B (green), α-actinin (red), and DAPI (blue) (A and B). (C, D) Respective gene expression levels were analyzed by qRT-PCR. Data are expressed as mean ± SEM. *P<0.05, **P<0.01. Statistical analysis was performed by the 2-tailed Student's t test.

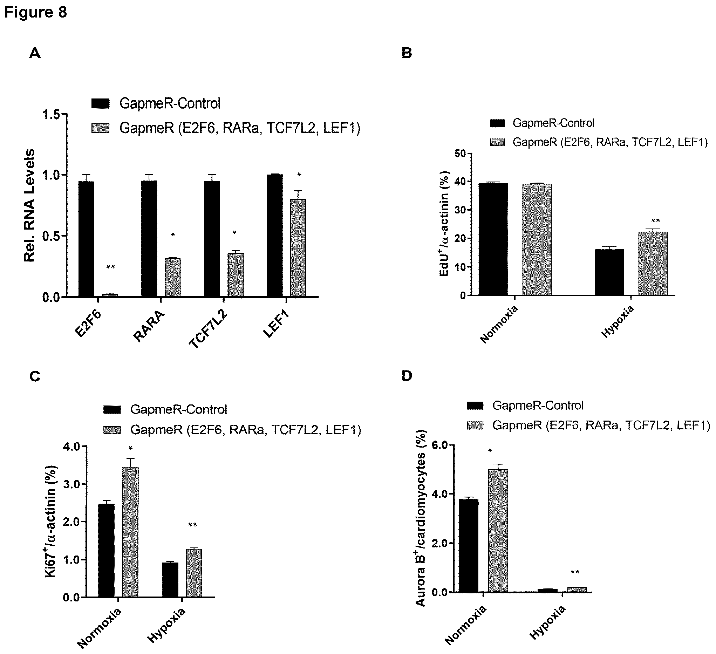

Figure 8 Mouse cardiomyocyte proliferation upon E2F6/RARa/TCF7L2/LEF1 knockdown
Mouse HL1 cells were treated with either GapmeR targeting E2F6, RARA, TCF7L2 and LEF1 or control GapmeR-control and then the cells were exposed to normoxia (20% O₂) or hypoxia (3% O₂) for 2 days. (A) The RNA expression levels were analyzed by qRT-PCR. (B) Quantification of the percentage of EdU⁺ cardiomyocytes. (C) Quantification of the percentage of Ki67⁺ cardiomyocytes. (D) Quantification of the percentage of Aurora B⁺ cardiomyocytes. Data are presented as mean ± SEM, * p < 0.05, ** p < 0.01.

### Examples

### Example 1: Proliferation on neonatal rat cardiomyocytes in vitro

To identify Transcription factors (TF) regulating cardiomyocyte proliferation, the inventors first cultured neonatal rat cardiomyocytes in varying glucose (Glc) concentrations (Figure 1A-1D) to determine the effects of changes in Glc levels in driving cardiomyocyte proliferation. In doing so, the inventors observed stark differences between 0 mM and Glc concentration at 2 mM and above (Fig. 1A-1D). The inventors reasoned that differential TF activation between 0mM Glc, and at 2mM Glc (and above) could prove critical in driving cardiomyocyte proliferation. In using the Cignal Transcription Factor Reporter Assay (Qiagen), the inventors identified a number of TF pathways differentially activated between 0mM Glc, and 2/3mM Glc (Figure 2).

### Example 2: Screening for inhibition of TFs that promote cardiomyocyte proliferation

Respective TF pathways encompass a number of individual TF and TFs isoforms. To identify specific TFs relevant and active in cardiomyocytes, the inventors cross-referenced individual TFs contained within the identified TF pathways with genomewide RNAseq and single cell-RNAseq datasets from public databases and repositories (including NCBI GEO, UCSC Genome Browser, etc.). In doing so, the inventors identified cardiac-relevant TFs as listed in Figure 3A and 3B. To knockdown the TFs in rat cardiomyocytes, the ON-TARGET SMARTpool siRNAs were used in the study.

Previous studies have attempted to induce cardiomyocyte cell-cycle re-entry through downregulation or upregulation of specific TFs. However, such approaches largely resulted in mild changes in cardiomyocyte proliferation. This is largely due to the fact that cardiomyocytes need to overcome a number of physical, morphological and structural barriers (pertaining primarily to the post-mitotic and terminal differentiation status of cardiomyocytes) for completion of cytokinesis and daughter cell formation. Due to the need for cooperative and concerted action of a number of distinct signaling pathways to overcome these barriers, the inventors employed the approach taken by Yamanaka and colleagues to identify pluripotency-inducing TFs (Takahashi et al., Cell, vol. 131(5), P861-872, 2007). Due to the similar necessity for combinatorial and concerted action of multiple signaling pathways for pluripotency induction of adult cells, Takahashi et al. established a screening methodology for the identification of combinatorial TFs function.

In taking a similar strategy the inventors identified which of the 34 candidate TFs were critical for cardiomyocyte proliferation. The neonatal rat cardiomyocytes (NRCs, P1) were transfected with all 34 siRNAs targeting the respective TFs together as a control, and examined the cardiomyocyte proliferation of withdraw of individual siRNA from the pool of 34 siRNAs by using EdU (5-Ethynyl-2'-deoxyuridine) incorporation (Figure 3A, 3B and 3C). The initial assessment for EdU was done within 3 days after transfection, and the rat cardiomyocytes were marked by sarcomeric α-actinin. Removal of individual siRNA targeting LEF1, TCF7L2, RARb, RARa, Myc, IRF1, Gli1, HSF1, NFYB, E2F7, E2F6 or E2F5 resulted in decreased numbers of cardiomyocytes in S-phase, as shown in Figure 3B and 3C. The RNA expression levels were detected by quantitative real time PCR (Figure 3D). These results indicate that LEF1, TCF7L2, RARb, RARa, Myc, IRF1, Gli1, HSF1, NFYB, E2F7, E2F6 and E2F5 play important roles in the DNA synthesis in rat cardiomyocytes.

To determine which of the 12 TFs were critical for cardiomyocyte cytokinesis, the inventors utilized Aurora B as marker. The isolated NRCs were transfected with all 12 siRNAs targeting the respective TFs together as a control, and examined the cardiomyocyte proliferation of withdraw of individual siRNA from the pool of 12 siRNAs by using Aurora B (Figure 4A and 4B). As shown in 4A and 4B, Removal of individual siRNA targeting LEF1, TCF7L2, RARa, NFYB, E2F7, E2F6 resulted in decreased numbers of cardiomyocyte cytokinesis. The RNA expression levels were detected by quantitative real time PCR (Figure 4C). These results indicate that LEF1, TCF7L2, RARa, NFYB, E2F7 and E2F6 play important roles in the cytokinesis in rat cardiomyocytes.

### Example 3: Combination knockdown of E2F6/RARa/TCF7L2/LEF1 or E2F7/NFYb/RARa/TCF7L2 induces cardiomyocyte proliferation in rat cardiomyocytes

To determine which combination could enhance cytokinesis, 63 conditions corresponding to combinations of the 6 candidate siRNAs targeting LEF1, TCF7L2, RARa, NFYB, E2F7 and E2F6 tested in 1-day-old (P1) and 7-day-old (P7) rat cardiomyocytes. Cardiomyocyte proliferation was assessed by co-localization of contractile ring and midbody Aurora B staining with sarcomeric α-actinin to marking cells in cytokinesis in P1 rat cardiomyocytes (Figure 5A and 5B) and P7 rat cardiomyocytes (Figure 5C and 5D). These results showed that 3 combinations (siE2F6, siRARa, siTCF7L2 and siLEF1; siE2F7, siNFYb, siRARa and siTCF7L2; siE2F6, siE2F7, siRARa, siTCF7L2 and siLEF1) have higher numbers of cardiomyocytes in telophase than other combinations. The inventors selected the specific combination of siE2F6, siRARa, siTCF7L2 and siLEF1; and siE2F7, siNFYb, siRARa and siTCF7L2 As shown in Figure 6A and 6B, knockdown of E2F6/RARa/TCF7L2/LEF1 or E2F7/NFYb/RARa/TCF7L2 remarkably increased cardiomyocytes proliferation by significantly increased Aurora B-positive cardiomyocytes in telophase. The gene expression levels were shown in Figure 6C and 6D. Furthermore, combination of siE2F6, siRARa, siTCF7L2 and siLEF1; or siE2F7, siNFYb, siRARa and siTCF7L2 increased Aurora B⁺ cardiomyocyte in Telophase in P7 rat cardiomyocytes, as shown in Figure 6E and 6F. The gene expression levels were shown in Figure 6G and 6H.

### Example 4: Combination knockdown of E2F6/RARa/TCF7L2/LEF1 or E2F7/NFYb/RARa/TCF7L2 induces cardiomyocyte proliferation in hiPSC-CMs

To test the combination in human cardiomyocytes, the inventors transfected siRNAs targeting E2F6/RARa/TCF7L2/LEF1, E2F7/NFYb/RARa/TCF7L2 or control scramble siRNA (siScr) in post-mitotic 40-day-old human induced pluripotent stem cell-derived cardiomyocytes (hiPSC-CMs), and then the hiPSC-CMs were exposed to normoxia (20% O₂) or hypoxia (3% O₂) for 2 days. As shown in Figure 7A and 7B, Furthermore, combination of siE2F6, siRARa, siTCF7L2 and siLEF1; or siE2F7, siNFYb, siRARa and siTCF7L2 increased Aurora B⁺ cardiomyocyte in Telophase in hiPSC-CMs. The gene expression levels were detected by qRT-PCR (Figure 7C and 7D). These results indicate that combination knockdown of E2F6/RARa/TCF7L2/LEF1 or E2F7/NFYb/RARa/TCF7L2 induces cardiomyocyte proliferation in hiPSC-CMs.

### Example 5: Combination knockdown of E2F6/RARa/TCF7L2/LEF1 induces cardiomyocyte proliferation in HL-1 cells

Next, the inventors determined if combination knockdown of E2F6/RARa/TCF7L2/LEF1 induces mouse cardiomyocyte proliferation in HL-1cells. The HL-1 cells were treated with either GapmeR targeting E2F6/RARa/TCF7L2/LEF1 or control GapmeR-control, and then the cells were exposed to Normoxia (20% O₂) and Hypoxia (3% O₂) for 2 days. The gene expression levels were detected by qRT-PCR (Figure 8A). As shown in Figure 8B, 8C and 8D, combination knockdown of E2F6/RARa/TCF7L2/LEF1 increased cardiomyocyte proliferation as determined by increased EdU⁺, Ki67⁺ and Aurora B⁺ cardiomyocytes. These data show that combination knockdown of E2F6/RARa/TCF7L2/LEF1 increased mouse cardiomyocyte proliferation in HL-1 cells.

### Materials and Methods

### siRNAs

All siRNAs were purchased from Dharmacon. A mix of ON-TARGETplus SMARTpool siRNAs directed against rat E2F6 (313978) sequences UGACAAAGAAAACGAAAGA (SEQ ID 009), CCGGAAGAGGCGUGUGUAU (SEQ ID 010), GGAUAGGAUCUGACCUGAA (SEQ ID 011), GACGAGUUGAUUAAAGAUU (SEQ ID 012); rat E2F7 (314818) sequences GCAUCUAUGACAUCGUAAA (SEQ ID 017), AGAAGAGCGAGGCCGCAAA (SEQ ID 018), CAACGGAGACAUCGGGAGA (SEQ ID 019), GGAUAAAUGUAUAUACGUG (SEQ ID 020); rat NFYb (253336) sequences AAUAAGUAGCUGUAACGUA (SEQ ID 021), GCAUGGUAACUUAGCCGAA (SEQ ID 022), AAUCUUGUGUACAUCGUAA (SEQ ID 023), CAUCGGAGGAAGUCAUUAU (SEQ ID 024); rat RARa (24705) sequences GCAAGUACACUACGAACAA (SEQ ID 001), CGAAUCUGCACGCGGUACA (SEQ ID 002), AAGACAAGUCAUCCGGCUA (SEQ ID 003), GCUCAAACCACUCCAUCGA (SEQ ID 004); rat TCF7L2 (679869) sequences GCACUUACCAGCUGACGUA (SEQ ID 005), GGAGAGGGAUUUAGCCGAU (SEQ ID 006), GCACACAUCGUUUCGAACA (SEQ ID 007), CAAAACAGCUCCUCCGAUU (SEQ ID 008); rat LEF1 (161452) sequences GGGCGAAUGUCGUAGCCGA (SEQ ID 013), CGUCACACAUCCCGUCAGA (SEQ ID 014), CGAAGAGGAGGGCGACUUA (SEQ ID 015), CAAUAUGAAUAGCGACCCA (SEQ ID 016);
human E2F6 (1876) sequences CAACGGACCUAUCGAUGUC (SEQ ID 165), UAGCAUAUGUGACCUAUCA (SEQ ID 166), GUAAGCAACUGAUGGCAUU (SEQ ID 167), GAACAGAUCGUCAUUGCAG (SEQ ID 168); human E2F7 (144455) sequences GCACACAUCGUGAGACGUU (SEQ ID 169), UGACUAACCUGCCGCUUUG (SEQ ID 170), CAAGGACGAUGCAUUUACA (SEQ ID 171), GGACUAUUCCGACCCAUUG (SEQ ID 172);
human NFYb (4801) sequences GAUAUUCUCUUUGCUAUGC (SEQ ID 173), GUAAGUGAGUUCAUCAGUU (SEQ ID 174), GAAUGUGUCAGGUGCAUAA (SEQ ID 175), UAUGCAAGCUCCAUGUUA (SEQ ID 176); human RARa (5914) sequences GCAAAUACACUACGAACAA (SEQ ID 177), CCAAGGAGUCUGUGAGAAA (SEQ ID 178), GAGCAGCAGUUCUGAAGAG (SEQ ID 179), GAACAACGUGUCUCUCUGC (SEQ ID 180);
human TCF7L2 (6934) sequences CAGCGAAUGUUUCCUAAAU (SEQ ID 181), CGAGACAAAUCCCGGGAAA (SEQ ID 182); ACACUUACCAGCCGACGUA (SEQ ID 183), GAUGUCGGCUCACUCCAUA (SEQ ID 184); human LEF1 (51176) sequences GCAAGAGACAAUUAUGGUA (SEQ ID 185), UGCCAAAUAUGAAUAACGA (SEQ ID 186), CGAUUUAGCUGACAUCAAG (SEQ ID 187), CGUCACACAUCCCAUCAGA (SEQ ID 188); An ON-TARGETplus non-targeting siRNA pool from Dharmacon served as a control.

### Isolation and maintenance of primary neonatal rat cardiomyocytes

Mated female Sprague Dawley rats were obtained from Janvier (Le Genest Saint-Isle, France). Primary neonatal rat cardiomyocytes (NRCs) were isolated from P1 and P7 rat pups by using the neonatal heart dissociation kit (Miltenyi Biotec) and the gentleMACS Dissociator according to the manufacturer's instruction. Isolated cells were pre-plated in plating medium (DMEM high glucose, M199 EBS (BioConcept), 10% horse serum, 5% fetal calf serum, 2% L-glutamine and 1% penicillin/streptomycin) for 1.5 h in 10 cm cell culture dishes (Nunc) at 37°C and 5% CO₂ at humidified atmosphere to get rid of fibroblasts as described previously [20]. NRCs were plated on Type-I bovine Collagen-coated (Advanced Biomatrix) plates or dishes in plating medium. 24 h after isolation of NRCs, the plating medium was changed to maintenance medium (DMEM high glucose, M199 EBS, 1% horse serum, 2% L-glutamine and 1% penicillin/streptomycin).

### Preparation and maintenance of human iPSC-CMs

Human induced pluripotent stem cells (hiPSCs) were purchased from Cellular Dynamics International (CMC-100-010-001) and cultured as recommended by the manufacturer. The human iPSC-derived cardiomyocytes (hiPSC-CMs) were reprogrammed using the STEMdiff^{™} Cardiomyocyte Differentiation Kit (STEMCELL Technologies) according to the manufacture's protocol. Briefly, human iPS cells were plated at cell density of 3.5×10⁵ cells/well on Matrigel coated 12-well-plates using mTeSR^{™} medium supplemented with 5uM ROCK inhibitor (Y-27632, STEMCELL Technologies) for 24 h. After 1 day (-1), the medium was replaced with fresh TeSR^{™} medium. To induce cardiac differentiation, the TeSR^{™} medium is replaced with Medium A (STEMdiff^{™} Cardiomyocyte Differentiation Basal Medium containing Supplement A) at day 0, Medium B (STEMdiff^{™} Cardiomyocyte Differentiation Basal Medium containing Supplement B) at day 2, Medium C (STEMdiff^{™} Cardiomyocyte Differentiation Basal Medium containing Supplement C) at day 4 and day 6. On day 8, medium was switched to STEMdiff^{™} Cardiomyocyte Maintenance Medium with full medium changes every 2 days, to promote further differentiation into mature cardiomyocyte cells. All experiments were performed in the hiPSC-CMs at day 40. Hypoxic condition was achieved by using the Hypoxia chamber and the hiPSC-CMs were cultured at 3% O₂ for 2 days.

### RNA isolation, reverse transcription and qRT-PCR

Samples were harvested in QIAzol Lysis Reagent (QIAGEN) and total miRNAs and total mRNAs were isolated with miRNA mini Kit (QIAGEN) according to the manufacturer's protocol. 200ng total RNAs were reverse transcript into cDNA using QuantiTect Reverse Transcription Kit (QIAGEN) according to the manufacturer's protocol. The Applied Biosystems StepOnePlus Real-Time PCR system (Applied Biosystems, CA, USA) with Fast SYBR○,R Green Master Mix (Thermo Fisher Scientific) were used for analysis. Gene expression levels were normalized against the housekeeping gene *HPRT1.* The following qRT-PCR primers were used: Rat: E2F6: 5' GTGGAGAACCTACTGCCATCA 3' (SEQ ID 137) and 5' GTGGCAACCTTGTTTAGGTCA' (SEQ ID 138) ; E2F7: 5' TCGCTATCCAAGCTACCCCT 3' (SEQ ID 139) and 5' CCGACCATGCCAACCATACT 3' (SEQ ID 140); RARa: 5' TGGCTCAAACCACTCCATCG 3' (SEQ ID 141) and 5' CGTCGGAAGAAGCCCTTACA 3' (SEQ ID 142); NFYb: 5' CTCTCGGCTTCGACAGCTAC 3' (SEQ ID 143) and 5' TCCATCTGTAGCAGACACGG 3' (SEQ ID 144); TCF7L2: 5' CGCACTTACCAGCTGACGTA 3' (SEQ ID 145) and 5' GTACACAGGCTGACCTTGCT 3' (SEQ ID 146); LEF1: 5' GGGACACTTCCATGTCCAGG 3' (SEQ ID 147) and 5' TTCACGTGCATTAGGTCGCT 3' (SEQ ID 148); HPRT: 5' CCTCCTCCGCCAGCTT 3' (SEQ ID 149) and 5' GTCATAACCTGGTTCATCATCACT (SEQ ID 150) 3'; Human: E2F6: 5' GGAGCACCAACGGACCTATC 3' (SEQ ID 151) and 5' CAGGGCCTTCTGGATGAGTG 3' (SEQ ID 152); E2F7: 5' CAGGCAGCCCAGACTAGATT 3' (SEQ ID 153) and 5' TATTGGAGTCTTCGGGGCCA 3' (SEQ ID 154); RARa: 5' CCTATGCTGGGTGGACTCTC 3' (SEQ ID 155) and 5' AGCAAGGCTTGTAGATGCGG 3' (SEQ ID 156); NFYb: 5' ATGCCATACCTCAAACGGGA 3' (SEQ ID 157) and 5' CCGTTTCTCTTGATGGCACCT 3' (SEQ ID 158); TCF7L2: 5' GGCAAGATGGAGGGCTCTTT 3' (SEQ ID 159) and 5' CGCAGAGTAATGTGTGCTGC 3' (SEQ ID 160); LEF1: 5' TATCCAGGCTGGTCTGCAAG 3' (SEQ ID 161) and 5' GCAGCTGTCATTCTTGGACC 3' (SEQ ID 162); HPRT5' CCTGGCGTCGTGATTAGTGAT 3' (SEQ ID 163) and 5' AGACGTTCAGTCCTGTCCATAA 3' (SEQ ID 164).

### Immunofluorescence staining

The NRCs were fixed with 4% paraformaldehyde (PFA)/PBS, the cells were permeabilized and incubated overnight at 4°C with primary antibodies against sarcomeric α-actinin (Sigma Aldrich), Aurora B (Abcam) diluted in 2% (v/v) HS/PBS. After 3 washes with PBS for 5 min, cells were incubated with 4',6-diamidino-2-phenylindole (DAPI; Thermo Fisher Scientific), AlexaFluor 555 anti-mouse (Thermo Fisher Scientific) and AlexaFluor 488 anti-rabbit (Thermo Fisher Scientific) secondary antibody for 1 h at room temperature. Dishes were mounted onto glass slides (Fisher Scientific) with a drop of ProLong Antifade (Thermo Fisher Scientific). Fluorescent images were acquired with the SP8 confocal microscopy (Leica) using a 40x magnification.

### Statistical Analysis

Data are represented as mean and error bars indicate the standard error of the mean (SEM). Two-tailed unpaired Student's t-tests (Excel) or one-way ANOVA analyses followed by either a Dunnett's multiple comparison post-test (multiple comparisons to a single control) or Bonferroni correction (multiple comparisons between different groups) were used as indicated in the respective figure legends. n.s. = not significant; *p < 0.05; **p < 0.01.

### Sequences of siRNAs

| | Gene Symbol | Sequence | SEQ ID NO |
|---|---|---|---|
| 1 | E2f1 | GUGCAGAAACGACGCAUCU | 025 |
| | E2f1 | CCAUGGAAGAAGACCGGUU | 026 |
| | E2f1 | GCUUAAAGGUUUUCCGAUC | 027 |
| | E2f1 | ACUCAGACAUCCAGCGCCU | 028 |
| 2 | E2f2 | AGACAGUGAUUGCGGUCAA | 029 |
| | E2f2 | GCAUCCAGCUCAUCCGCAA | 030 |
| | E2f2 | CCUACUACACUUCGCUUUA | 031 |
| | E2f2 | CAAGAUUGGAAGUGCCGGA | 032 |
| 3 | E2f3 | CAUAGCGACUGCUCGGUUU | 033 |
| | E2f3 | UCAAUAGAGAGCCUACAAA | 034 |
| | E2f3 | CUGUUAUAGUUGUGAAAGC | 035 |
| | E2f3 | AGAGGAGGAUCUACGACAU | 036 |
| 4 | E2f4 | CCGGGAGAUUGCCGACAAA | 037 |
| | E2f4 | GGUAUUGGUCUGAUCGAGA | 038 |
| | E2f4 | GGAAUGUCGCAGAGGACGU | 039 |
| | E2f4 | ACUCCAAGCCGGCACGAGA | 040 |
| 5 | E2f5 | UGAUAUCACCAACGUCUUA | 041 |
| | E2f5 | GAUCUAGAAUUGAAGGAAA | 042 |
| | E2f5 | GCAGUUGCUUUAAUGGUGA | 043 |
| | E2f5 | AGACAUUCACUGCCAUAUA | 044 |
| 6 | E2f6 | UGACAAAGAAAACGAAAGA | 009 |
| | E2f6 | CCGGAAGAGGCGUGUGUAU | 010 |
| | E2f6 | GGAUAGGAUCUGACCUGAA | 011 |
| | E2f6 | GACGAGUUGAUUAAAGAUU | 012 |
| 7 | E2f7 | GCAUCUAUGACAUCGUAAA | 017 |
| | E2f7 | AGAAGAGCGAGGCCGCAAA | 018 |
| | E2f7 | CAACGGAGACAUCGGGAGA | 019 |
| | E2f7 | GGAUAAAUGUAUAUACGUG | 020 |
| 8 | Nfya | ACACAGAUCAUCCGAGUUU | 045 |
| | Nfya | CCAAACAGUAUCACCGCAU | 046 |
| | Nfya | GGACAAGGCCAAACUAUCA | 047 |
| | Nfya | UGGAAGAAGAGCCGUUGUA | 048 |
| 9 | Nfyb | AAUAAGUAGCUGUAACGUA | 021 |
| | Nfyb | GCAUGGUAACUUAGCCGAA | 022 |
| | Nfyb | AAUCUUGUGUACAUCGUAA | 023 |
| | Nfyb | CAUCGGAGGAAGUCAUUAU | 024 |
| 10 | Nfyc | GAGGAUAACAAGCGUCGUA | 049 |
| | Nfyc | CCGGCCAGUUGCAGUAUAU | 050 |
| | Nfyc | ACGUGUGGUUGAAGAAAUA | 051 |
| | Nfyc | GCUAUGUGUCCAAUGCUAU | 052 |
| 11 | Yy1 | CAUCUUAACACACGCUAAA | 053 |
| | Yy1 | AGGGAUAACUCUGCUAUGA | 054 |
| | Yy1 | GGGCGACACCCUCUACAUU | 055 |
| | Yy1 | GCGCCGACCCGGGUAAUAA | 056 |
| 12 | Hsf1 | CCUCAUAGCCCUCGGGUAC | 057 |
| | Hsf1 | GAGAAGUGCCUCAGCGUAG | 058 |
| | Hsf1 | GAAUAGUAACGCCGACUCA | 059 |
| | Hsf1 | GGCCCAUAAUCUCCGAUAU | 060 |
| 13 | Gli1 | GCACUAAGGCUCUAGGUAC | 061 |
| | Gli1 | CAGCUAGAGUACAACGGUU | 062 |
| | Gli1 | CUGCAAACCGUAAUCCGUA | 063 |
| | Gli1 | CCAACAUGGCAGUCGGUAA | 064 |
| 14 | Hif1a | UGAGAGAAAUGCUUACACA | 065 |
| | Hif1a | GGAAACGAGUGAAAGGAUA | 066 |
| | Hif1a | UUACUGAGUUGAUGGGUUA | 067 |
| | Hif1a | CUGAUAACGUGAACAAAUA | 068 |
| 15 | Irf1 | CGAGAUAGGCAUACAGCGU | 069 |
| | Irf1 | CCUACCAGACAUCGAGGAA | 070 |
| | Irf1 | AGGAAGAUGGAUUGGCGUU | 071 |
| | Irf1 | AGGAGGAACCAGAGAUCGA | 072 |
| 16 | Klf4 | CGACUAACCGUUGGCGAGA | 073 |
| | Klf4 | UCUAAUAGCCUAAACGAUG | 074 |
| | Klf4 | CCGAGGAGUUCAACGAUCU | 075 |
| | Klf4 | UGACCAGGCACUACCGCAA | 076 |
| 17 | Myc | GUGCAUAAACUGACCGGAA | 077 |
| | Myc | CCUCAAAGUUAAGGCAUAA | 078 |
| | Myc | AGAAACGAGCUGAAGCGUA | 079 |
| | Myc | CUUACCAGGCUGCGCGCAA | 080 |
| 18 | Nanog | GAAUUCUCCAGGCGAAAUA | 081 |
| | Nanog | GGGAAAGCCAGGCGCAUUU | 082 |
| | Nanog | CCAGUGAUUUGGAGGCGAA | 083 |
| | Nanog | UAACGAUGGGCUUCGGAGA | 084 |
| 19 | Nfkb1 | CGAGAUAAUGACAGCGUGU | 085 |
| | Nfkb1 | CUGCAAAGGUUAUCGUUCA | 086 |
| | Nfkb1 | AAUACUAGAGCAACCGAAA | 087 |
| | Nfkb1 | CGACGAUCCUUUCGGAACU | 088 |
| 20 | Pax6 | GGGUAAGAUAUGAAUUCGA | 089 |
| | Pax6 | GUAGAGUGUGUCUUCGAUA | 090 |
| | Pax6 | GCGUACAGAAGGCACGGUA | 091 |
| | Pax6 | AUGGAGAAGACUCGGAUGA | 092 |
| 21 | Nfatc1 | ACACAAUUCGCCUGCGUUA | 093 |
| | Nfatc1 | GUAAUAUAUCUGUCCGAUU | 094 |
| | Nfatc1 | CCAAAGGGCAGCAUCCGAA | 095 |
| | Nfatc1 | GGACUUAGCCCUCUGCCGA | 096 |
| 22 | Nfatc2 | CGGUUCGGAGAGUCGGAUA | 097 |
| | Nfatc2 | AAGAAGAACCGAUCGCACA | 098 |
| | Nfatc2 | UUUCCAUCCUCUUCGAUUA | 099 |
| | Nfatc2 | UAGCUGAUGUCCACGGUGA | 100 |
| 23 | Nfatc3 | GAACCGUCCUACCGGGAAU | 101 |
| | Nfatc3 | GAAUGAAGCUGCUGCACGA | 102 |
| | Nfatc3 | AAAGAAUACUAGAGUCCGA | 103 |
| | Nfatc3 | CAGAUAUAGAACUUCGAAA | 104 |
| 24 | Pgr | GUUCCCAGUUUACGGCGAA | 105 |
| | Pgr | CUAUAUCUCACCCGUGUUA | 106 |
| | Pgr | CUAUUUAUGUGCCGGAAGA | 107 |
| | Pgr | GGAACUUACACAUCGAUGA | 108 |
| 25 | Rara | GCAAGUACACUACGAACAA | 001 |
| | Rara | CGAAUCUGCACGCGGUACA | 002 |
| | Rara | AAGACAAGUCAUCCGGCUA | 003 |
| | Rara | GCUCAAACCACUCCAUCGA | 004 |
| 26 | Rarb | CGGGAUAAGAACUGCGUUA | 109 |
| | Rarb | GGUAAAUACACCACGAAUU | 110 |
| | Rarb | AGGACAAGUCAUCGGGCUA | 111 |
| | Rarb | CCGCAGCAUCAGCGCCAAA | 112 |
| 27 | Rarg | GGGCUGACCCUGAACCGAA | 113 |
| | Rarg | GUAAGGAAUGACCGGAACA | 114 |
| | Rarg | CGACAAGUCUUCCGGCUAC | 115 |
| | Rarg | CUGCAAAAGUGUUUCGAAG | 116 |
| 28 | Sox2 | CGGAAAACCAAGACGCUCA | 117 |
| | Sox2 | GCUCUGUGGUCAAGUCCGA | 118 |
| | Sox2 | AUGAAGGAGCACCCGGAUU | 119 |
| | Sox2 | CAGUACAACUCCAUGACCA | 120 |
| 29 | Smad2 | UCAGGUGUCUCAUCGGAAA | 121 |
| | Smad2 | ACUCGAAUGUGCACCAUAA | 122 |
| | Smad2 | GCCGAGUGCCUAAGUGAUA | 123 |
| | Smad2 | GGAAUUGAGCCACAGAGUA | 124 |
| 30 | Smad3 | GCACAGCCACCAUGAGUUA | 125 |
| | Smad3 | GGAUCGAGCUACACCUGAA | 126 |
| | Smad3 | GUGAACACCAAGUGCAUUA | 127 |
| | Smad3 | UCACUGAUCCCUCCAAUUC | 128 |
| 31 | Smad4 | CGGAAGGACAUUCGAUUCA | 129 |
| | Smad4 | GGACAUUACUGGCCGGUUC | 130 |
| | Smad4 | GCAGGUGGCUGGUCGGAAA | 131 |
| | Smad4 | GCGCAUCCACGGAGACGUA | 132 |
| 32 | Tcf7 | AAGACCAGCUCACGUGGAA | 133 |
| | Tcf7 | GCGAGGAACAGGACGAUAA | 134 |
| | Tcf7 | AGUCCAUGGAGAAGCCGAA | 135 |
| | Tcf7 | CUAACAGCUUUGCGUGCUA | 136 |
| 33 | Tcf7l2 | GCACUUACCAGCUGACGUA | 005 |
| | Tcf7l2 | GGAGAGGGAUUUAGCCGAU | 006 |
| | Tcf7l2 | GCACACAUCGUUUCGAACA | 007 |
| | Tcf7l2 | CAAAACAGCUCCUCCGAUU | 008 |
| 34 | Lef1 | GGGCGAAUGUCGUAGCCGA | 013 |
| | Lef1 | CGUCACACAUCCCGUCAGA | 014 |
| | Lef1 | CGAAGAGGAGGGCGACUUA | 015 |
| | Lef1 | CAAUAUGAAUAGCGACCCA | 016 |

## Claims

1. A combination of nucleic acid agents, wherein each of said nucleic acid agents is capable of specifically inhibiting the expression of a target protein, whereby the combination of nucleic acid agents is capable of inhibiting the expression of a combination of target proteins within a cell,
wherein said combination of target proteins is
a. RARa, and
b. TCF7L2, and
c. E2F6 and LEF1, or
E2F7 and NFYb;
for use in treatment or prevention of heart disease.

2. The combination of nucleic acid agents for use in treatment or prevention of heart disease according to claim 1, wherein said combination of target proteins is
a. RARa, and
b. TCF7L2, and
c. E2F6, and
d. LEF1.

3. The combination of nucleic acid agents for use in treatment or prevention of heart disease according to claim 1 to 2, wherein each nucleic acid agent is selected independently from an antisense oligonucleotide, an siRNA, an shRNA, an sgRNA and an miRNA, particularly each nucleic acid agent is an siRNA.

4. The combination of nucleic acid agents for use in treatment or prevention of heart disease according to any one of the preceding claims, wherein each nucleic acid agent comprises, or essentially consists of, a sequence selected from the sequences SEQ ID NO 165-188.

5. The combination of nucleic acid agents for use in treatment or prevention of heart disease according to any one of the preceding claims, wherein one of said nucleic acid agents comprises or essentially consists of,
particularly wherein all of said nucleic acid agents comprise or essentially consist of,
one or several locked nucleic acid (LNA) and/or peptide nucleic acid (PNA) moieties, particularly wherein said nucleic acid agents essentially consist of locked nucleic acid (LNA) moieties.

6. The combination of nucleic acid agents for use in treatment or prevention of heart disease according to any one of the preceding claims, wherein one of said nucleic acid agents comprises,
particularly wherein all of said nucleic acid agents comprise one or several thiophosphate linkages.

7. The combination of nucleic acid agents for use in treatment or prevention of heart disease according to any one of the preceding claims, wherein one of said nucleic acid agents is,
particularly wherein all of said nucleic acid agents are,
expressed intracellularly from an exogenous (transgene) expression construct comprising a sequence encoding a hybridizing sequence of nucleotides under control of a promoter operable in a human myocardial cell.

8. An expression construct, or a plurality of expression constructs, encoding, under control of a promoter operable in a human myocardial cell, a combination of nucleic acid agents, wherein each of said nucleic acid agents is capable of specifically inhibiting the expression of a target protein, thereby inhibiting the expression of a combination of target proteins, wherein said combination of target proteins is
a. RARa, and
b. TCF7L2, and
c. E2F6 and LEF1, or
E2F7 and NFYb
for use in treatment or prevention of heart disease.

9. The plurality of expression constructs for use in treatment or prevention of heart disease according to claim 8, wherein each and all of said expression constructs are comprised within a single nucleic acid molecule.

10. The combination of nucleic acid agents, or the plurality of expression constructs, for use in treatment or prevention of heart disease according to any one of the preceding claims, wherein said nucleic acid agents or said expression construct are bound to a nanoparticle or encapsulated by a particulate structure selected from a virus and a liposome.

11. The expression construct, or the plurality of expression constructs, for use in treatment or prevention of heart disease according to any one of claims 8 to 10 wherein said promoter is a heart-tissue-specific RNA Polymerase 2 promoter, particularly a promoter selected from Myosin Light Chain 2v (MLC2v) promoter, cardiac Troponin T (cTnT) promoter, Myosin Heavy Chain alpha (MHCa) promoter, and Titin (Ttn) promoter.

12. The combination of nucleic acid agents, or the plurality of expression constructs, for use in treatment or prevention of heart disease according to any one of the preceding claims, wherein said heart disease is **characterized by** a loss of cardiomyocytes, particularly said heart disease is heart failure **characterized by** a loss of cardiomyocytes, more particularly said heart failure is a result of myocardial infarction, stenosis, congenital disease, inflammation and/or sepsis.

13. The combination of nucleic acid agents, or the plurality of expression constructs, for use in treatment or prevention of heart disease according to any one of the preceding claims, wherein said heart disease is **characterized by** hypoxia of cardiomyocytes.

14. A pharmaceutical composition for use in treatment or prevention of heart disease comprising said combination of nucleic acid agents, or said plurality of expression constructs, according to any one of the preceding claims.

15. The pharmaceutical composition for use in treatment or prevention of heart disease according to claim 14, wherein the pharmaceutical composition is administered to the heart.

## Patentansprüche

1. Eine Kombination von Nukleinsäure-Agenzien, wobei jeder der genannten Nukleinsäure-Agenzien in der Lage ist, die Expression eines Zielproteins spezifisch zu inhibieren, wodurch die Kombination von Nukleinsäure-Agenzien in der Lage ist, die Expression einer Kombination von Zielproteinen innerhalb einer Zelle zu inhibieren,
wobei die genannte Kombination von Zielproteinen ist
a. RARa und
b. TCF7L2 und
c. E2F6 und LEF1 oder
E2F7 und NFYb;
zur Verwendung bei der Behandlung oder Prävention von Herzerkrankungen.

2. Die Kombination von Nukleinsäure-Agenzien zur Verwendung bei der Behandlung oder Prävention von Herzerkrankungen nach Anspruch 1, wobei die genannte Kombination von Zielproteinen ist
a. RARa und
b. TCF7L2 und
c. E2F6 und
d. LEF1.

3. Die Kombination von Nukleinsäure-Agenzien zur Verwendung bei der Behandlung oder Prävention von Herzerkrankungen nach Anspruch 1 bis 2, wobei jedes Nukleinsäure-Agens unabhängig ausgewählt ist aus einem Antisense-Oligonukleotid, einer siRNA, einer shRNA, einer sgRNA und einer miRNA, insbesondere wobei jedes Nukleinsäure-Agens eine siRNA ist.

4. Die Kombination von Nukleinsäure-Agenzien zur Verwendung bei der Behandlung oder Prävention von Herzerkrankungen nach einem der vorstehenden Ansprüche, wobei jedes Nukleinsäure-Agens eine Sequenz ausgewählt aus den Sequenzen SEQ ID NO 165-188 umfasst oder im Wesentlichen daraus besteht.

5. Die Kombination von Nukleinsäure-Agenzien zur Verwendung bei der Behandlung oder Prävention von Herzerkrankungen nach einem der vorstehenden Ansprüche, wobei einer der genannten Nukleinsäure-Agenzien eine oder mehrere Locked-Nucleic-Acid-(LNA)- und/oder Peptid-Nukleinsäure-(PNA)-Einheiten umfasst oder im Wesentlichen daraus besteht,
insbesondere wobei alle der genannten Nukleinsäure-Agenzien eine oder mehrere Locked-Nucleic-Acid-(LNA)- und/oder Peptid-Nukleinsäure-(PNA)-Einheiten umfassen oder im Wesentlichen daraus bestehen,
insbesondere wobei die genannten Nukleinsäure-Agenzien im Wesentlichen aus Locked-Nucleic-Acid-(LNA)-Einheiten bestehen.

6. Die Kombination von Nukleinsäure-Agenzien zur Verwendung bei der Behandlung oder Prävention von Herzerkrankungen nach einem der vorstehenden Ansprüche, wobei einer der genannten Nukleinsäure-Agenzien eine oder mehrere Thiophosphatbindungen umfasst,
insbesondere wobei alle der genannten Nukleinsäure-Agenzien eine oder mehrere Thiophosphatbindungen umfassen.

7. Die Kombination von Nukleinsäure-Agenzien zur Verwendung bei der Behandlung oder Prävention von Herzerkrankungen nach einem der vorstehenden Ansprüche, wobei einer der genannten Nukleinsäure-Agenzien intrazellulär exprimiert ist aus einem exogenen (Transgen-)Expressionskonstrukt, das eine Sequenz umfasst, die eine hybridisierende Nukleotidsequenz codiert, unter der Kontrolle eines in einer menschlichen Myokardzelle wirksamen Promotors,
insbesondere wobei alle der genannten Nukleinsäure-Agenzien intrazellulär exprimiert sind aus einem exogenen (Transgen-)Expressionskonstrukt, das eine Sequenz umfasst, die eine hybridisierende Nukleotidsequenz codiert, unter der Kontrolle eines in einer menschlichen Myokardzelle wirksamen Promotors.

8. Ein Expressionskonstrukt oder eine Mehrzahl von Expressionskonstrukten, die unter der Kontrolle eines in einer menschlichen Myokardzelle wirksamen Promotors eine Kombination von Nukleinsäure-Agenzien codieren, wobei jeder der genannten Nukleinsäure-Agenzien in der Lage ist, die Expression eines Zielproteins spezifisch zu inhibieren, wodurch die Expression einer Kombination von Zielproteinen inhibiert wird, wobei die genannte Kombination von Zielproteinen ist
a. RARa und
b. TCF7L2 und
c. E2F6 und LEF1 oder
E2F7 und NFYb
zur Verwendung bei der Behandlung oder Prävention von Herzerkrankungen.

9. Die Mehrzahl von Expressionskonstrukten zur Verwendung bei der Behandlung oder Prävention von Herzerkrankungen nach Anspruch 8, wobei jedes und alle der genannten Expressionskonstrukte innerhalb eines einzelnen Nukleinsäuremoleküls enthalten sind.

10. Die Kombination von Nukleinsäure-Agenzien oder die Mehrzahl von Expressionskonstrukten zur Verwendung bei der Behandlung oder Prävention von Herzerkrankungen nach einem der vorstehenden Ansprüche, wobei die genannten Nukleinsäure-Agenzien oder das genannte Expressionskonstrukt an ein Nanopartikel gebunden oder von einer partikulären Struktur, ausgewählt aus einem Virus und einem Liposom, eingekapselt sind.

11. Das Expressionskonstrukt oder die Mehrzahl von Expressionskonstrukten zur Verwendung bei der Behandlung oder Prävention von Herzerkrankungen nach einem der Ansprüche 8 bis 10, wobei der genannte Promotor ein herzspezifischer RNA-Polymerase-II-Promotor ist, insbesondere ein Promotor ausgewählt aus dem Myosin-Light-Chain-2v-(MLC2v)-Promotor, dem kardialen Troponin-T-(cTnT)-Promotor, dem Myosin-Heavy-Chain-alpha-(MHCa)-Promotor und dem Titin-(Ttn)-Promotor.

12. Die Kombination von Nukleinsäure-Agenzien oder die Mehrzahl von Expressionskonstrukten zur Verwendung bei der Behandlung oder Prävention von Herzerkrankungen nach einem der vorstehenden Ansprüche, wobei die genannte Herzerkrankung durch einen Verlust von Kardiomyozyten gekennzeichnet ist, insbesondere wobei die genannte Herzerkrankung Herzinsuffizienz ist, die durch einen Verlust von Kardiomyozyten gekennzeichnet ist, noch insbesondere wobei die genannte Herzinsuffizienz eine Folge von Myokardinfarkt, Stenose, angeborener Erkrankung, Entzündung und/oder Sepsis ist.

13. Die Kombination von Nukleinsäure-Agenzien oder die Mehrzahl von Expressionskonstrukten zur Verwendung bei der Behandlung oder Prävention von Herzerkrankungen nach einem der vorstehenden Ansprüche, wobei die genannte Herzerkrankung durch Hypoxie von Kardiomyozyten gekennzeichnet ist.

14. Eine pharmazeutische Zusammensetzung zur Verwendung bei der Behandlung oder Prävention von Herzerkrankungen, umfassend die genannte Kombination von Nukleinsäure-Agenzien oder die genannte Mehrzahl von Expressionskonstrukten nach einem der vorstehenden Ansprüche.

15. Die pharmazeutische Zusammensetzung zur Verwendung bei der Behandlung oder Prävention von Herzerkrankungen nach Anspruch 14, wobei die pharmazeutische Zusammensetzung dem Herzen verabreicht wird.

## Revendications

1. Combinaison d'agents d'acide nucléique, dans laquelle chacun desdits agents d'acide nucléique est capable d'inhiber spécifiquement l'expression d'une protéine cible, moyennant quoi la combinaison d'agents d'acide nucléique est capable d'inhiber l'expression d'une combinaison de protéines cibles au sein d'une cellule,
dans laquelle ladite combinaison de protéines cibles est
a. RARa, et
b. TCF7L2, et
c. E2F6 et LEF1, ou
E2F7 et NFYb ;
destinée à être utilisée dans le traitement ou la prévention de maladie cardiaque.

2. Combinaison d'agents d'acide nucléique destinée à être utilisée dans le traitement ou la prévention de maladie cardiaque selon la revendication 1, dans laquelle ladite combinaison de protéines cibles est
a. RARa, et
b. TCF7L2, et
c. E2F6, et
d. LEF1.

3. Combinaison d'agents d'acide nucléique destinée à être utilisée dans le traitement ou la prévention de maladie cardiaque selon les revendications 1 à 2, dans laquelle chaque agent d'acide nucléique est sélectionné indépendamment parmi un oligonucléotide antisens, un ARNsi, un ARNsh, un ARNsg et un ARNmi, notamment chaque agent d'acide nucléique est un ARNsi.

4. Combinaison d'agents d'acide nucléique destinée à être utilisée dans le traitement ou la prévention de maladie cardiaque selon l'une quelconque des revendications précédentes, dans laquelle chaque agent d'acide nucléique comprend, ou est essentiellement constitué de, une séquence sélectionnée parmi les séquences SEQ ID N° 165 à 188.

5. Combinaison d'agents d'acide nucléique destinée à être utilisée dans le traitement ou la prévention de maladie cardiaque selon l'une quelconque des revendications précédentes, dans laquelle un desdits agents d'acide nucléique comprend ou est essentiellement constitué de,
notamment dans lequel l'ensemble desdits agents d'acide nucléique comprennent ou sont essentiellement constitués de,
un ou plusieurs groupes caractéristiques d'acide nucléique verrouillé (LNA) et/ou d'acide nucléique peptidique (PNA), notamment dans lequel lesdits agents d'acide nucléique sont essentiellement constitués de groupes caractéristiques d'acide nucléique verrouillé (LNA).

6. Combinaison d'agents d'acide nucléique destinée à être utilisée dans le traitement ou la prévention de maladie cardiaque selon l'une quelconque des revendications précédentes, dans laquelle un desdits agents d'acide nucléique comprend,
notamment dans lequel l'ensemble desdits agents d'acide nucléique comprennent un ou plusieurs liens thiophosphate.

7. Combinaison d'agents d'acide nucléique destinée à être utilisée dans le traitement ou la prévention de maladie cardiaque selon l'une quelconque des revendications précédentes, dans laquelle un desdits agents d'acide nucléique est,
notamment dans lequel l'ensemble desdits agents d'acide nucléique sont,
exprimé(s) par voie intracellulaire à partir d'une construction d'expression exogène (transgène) comprenant une séquence codant pour une séquence d'hybridation de nucléotides sous le contrôle d'un promoteur pouvant être opéré dans une cellule myocardique humaine.

8. Construction d'expression, ou pluralité de constructions d'expression, codant pour une combinaison d'agents d'acide nucléique sous le contrôle d'un promoteur pouvant être opéré dans une cellule myocardique humaine, dans laquelle chacun desdits agents d'acide nucléique est capable d'inhiber spécifiquement l'expression d'une protéine cible, inhibant de ce fait l'expression d'une combinaison de protéines cibles, dans laquelle ladite combinaison de protéines cibles est
a. RARa, et
b. TCF7L2, et
c. E2F6 et LEF1, ou
E2F7 et NFYb
destinée à être utilisée dans le traitement ou la prévention de maladie cardiaque.

9. Pluralité de constructions d'expression destinée à être utilisée dans le traitement ou la prévention de maladie cardiaque selon la revendication 8, dans laquelle chacune et l'ensemble desdites constructions d'expression sont comprises au sein d'une molécule d'acide nucléique unique.

10. Combinaison d'agents d'acide nucléique, ou pluralité de constructions d'expression, destinée à être utilisée dans le traitement ou la prévention de maladie cardiaque selon l'une quelconque des revendications précédentes, dans laquelle lesdits agents d'acide nucléique ou ladite construction d'expression sont lié(e)s à une nanoparticule ou encapsulé(e)s par une structure particulaire sélectionnée parmi un virus et un liposome.

11. Construction d'expression, ou pluralité de constructions d'expression, destinée à être utilisée dans le traitement ou la prévention de maladie cardiaque selon l'une quelconque des revendications 8 à 10, dans laquelle ledit promoteur est un promoteur d'ARN Polymérase 2 spécifique au tissu cardiaque, notamment un promoteur sélectionné parmi le promoteur de chaîne légère de myosine 2v (MLC2v), le promoteur de troponine cardiaque T (cTnT), le promoteur de chaîne lourde alpha de myosine (MHCa) et le promoteur de titine (Ttn).

12. Combinaison d'agents d'acide nucléique, ou pluralité de constructions d'expression, destinée à être utilisée dans le traitement ou la prévention de maladie cardiaque selon l'une quelconque des revendications précédentes, dans laquelle ladite maladie cardiaque est **caractérisée par** une perte de cardiomyocytes, en particulier ladite maladie cardiaque est l'insuffisance cardiaque **caractérisée par** une perte de cardiomyocytes, plus particulièrement ladite insuffisance cardiaque est un résultat d'infarctus du myocarde, de sténose, de maladie congénitale, d'inflammation et/ou de sepsie.

13. Combinaison d'agents d'acide nucléique, ou pluralité de constructions d'expression, destinée à être utilisée dans le traitement ou la prévention de maladie cardiaque selon l'une quelconque des revendications précédentes, dans laquelle ladite maladie cardiaque est **caractérisée par** l'hypoxie de cardiomyocytes.

14. Composition pharmaceutique destinée à être utilisée dans le traitement ou la prévention de maladie cardiaque comprenant ladite combinaison d'agents d'acide nucléique, ou ladite pluralité de constructions d'expression, selon l'une quelconque des revendications précédentes.

15. Composition pharmaceutique destinée à être utilisée dans le traitement ou la prévention de maladie cardiaque selon la revendication 14, dans laquelle la composition pharmaceutique est administrée au coeur.
